# EUROPEAN PATENT APPLICATION

(11) **EP 3 070 083 A1**
(43) Date of publication of application: **21.09.2016**
(21) Application number: 15159936.2
(22) Date of filing: 19.03.2015
(51) Int. Cl.: C07D 333/22, C07B 37/04, C07B 53/00, C07F 17/02, C07F 3/06, C07D 213/14, C07D 213/16, C07F 7/18, C07D 307/42

(54) **Organozinc reagents and processes for preparing and using the same**

(71) Applicant: Ludwig-Maximilians-Universität München, 80539 München (DE)
(72) Inventor: Knochel, Paul, 81479 München (DE); Ellwart, Mario, 80333 München (DE)
(74) Representative: Vossius & Partner Patentanwälte Rechtsanwälte mbB

(57) **Abstract**

Provided is an organozinc composition comprising (a) an organozinc complex carrying an allylic ligand, (b) a magnesium component and (c) a lithium component. The organozinc composition is useful as a synthetic intermediate with excellent reactivity and selectivity, and can be stored for extended periods of time without loss of activity. Moreover, a process for the preparation of the organozinc composition and synthetic procedures making use of the organozinc composition are provided.

## Description

The present invention relates to organozinc compositions comprising an organozinc complex carrying an allylic ligand, and defined magnesium and lithium components. Moreover, a process for the preparation of the organozinc composition and synthetic procedures making use of the organozinc composition are provided.

Organozinc reagents have found numerous applications in organic synthesis, in particular for addition reactions to carbonyl compounds to prepare allylic alcohols, and in Negishi cross-coupling reactions (e.g. P. Knochel et al. in Handbook of Functionalized Organometallics (Ed.: P. Knochel), Wiley-VCH, Weinheim 2005, pp. 251-333). However, many functionalized zinc reagents are highly sensitive to moisture and air, and their practical applicability as synthetic tools in the laboratory and on an industrial scale is significantly limited. In particular for use in medicinal chemistry, the need for synthetic reagents and building blocks for chemical synthesis which can be conveniently handled is large. For example, for the development of new active agents and for the provision of compound libraries to be screened, reagents are necessary which are characterized by a high stability. In this context, the preparation of solid aryl, heteroaryl and benzylzinc reagents was recently reported that are air stable but still display a high reactivity for forming new carbon-carbon bonds (cf. S. Bernhardt et al., Angew. Chem. Int. Ed. 2011, 50, 9205; C. I. Stathakis et al., Angew. Chem. Int. Ed. 2012, 51, 9428; C. I. Stathakis et al., Org. Lett. 2013, 15, 1302; J. R. Colombe et al., Org. Lett. 2013, 15, 5754; S. M. Manolikakes et al., Chem. Eur. J. 2014, 20, 12289; A. Hernán-Gómez et al., Angew. Chem., Int. Ed. 2014, 53, 2706).

Allylic organometallics are an important class of organometallic reagents due to their enhanced reactivity compared to the corresponding alkyl, aryl or even benzylic organometallics (cf. M. Yasuda et al., J. Am. Chem. Soc. 2002, 124, 13442; A. N. Thadani et al., Org. Lett. 2002, 4, 3827; G. Courtois et al., Tetrahedron Lett. 1985, 26, 1027; I. Mareket al., Chem. Commun. 2007, 1683). For example, allylic magnesium halides are much more reactive than all other classes of Grignard reagents (cf. D. F. Taber et al., J. Org. Chem. 1997, 62, 9342; Y. Yamamoto et al., Chem. Rev. 1993, 93, 2207). This behavior can be explained by the higher ionic character of the allylic C-metal bond. Although allylic derivatives of most main-group elements have been reported (e.g. M. Schlosser et al., Tetrahedron 1993, 49, 10175; Y. Yamamoto et al., J. Am. Chem. Soc. 1980, 102, 7107) allylic zinc reagents play an important role as useful reagents in synthetic organic chemistry since they display not only a high reactivity but are at the same time compatible with a range of functional groups including an ester (cf. Y. A. Dembéléa et al., Tetrahedron: Asymmetry, 1992, 3, 351; V. Nyzam et al., Tetrahedron: Asymmetry, 1996, 7, 1835; J. Villieras et al., Synthesis, 1982, 924) or a cyano function (cf. H. Ren et al., J. Am. Chem. Soc. 2007, 129, 5376; M. D. Helm et al., Chem. Commun. 2008, 1916; C. Sämann et al., Synthesis 2013, 1870). In addition, allylic zinc reagents can be prepared by an insertion of commercial zinc dust to the corresponding allylic bromide (e.g. M. Bellassoued et al., J. Organomet. Chem. 1979, 166, 1; F. Dardoize et al., Bull. Soc. Chim. Fr. 1976, 9, 1561; G. Courtoiset al., J. Organomet. Chem. 1974, 72, 309). However, due to their enhanced reactivity, the provision of stable, in particular stable solid allylzinc reagents that can be handled and/or stored in a satisfactory and convenient manner remains problematic.

In the context of the present invention, a new synthetic approach for the preparation of allylzinc reagents was found, which makes these reagents available in a stable, in particular solid form, and which is compatible with diverse substituted allyl ligands. This is remarkable e.g. since the synthesis of substituted allylzinc compounds is frequently accompanied by side-reactions such as homo-coupling of the target compounds. The solid allylzinc reagents in accordance with the invention can not only be handled, e.g. weighed, in a convenient manner, but also remain active over an extended period of time, e.g. weeks or months in an inert gas atmosphere and can be stored accordingly.

Thus, the invention provides, in a first aspect, an organozinc composition comprising:
(a) an organozinc complex, wherein a zinc atom carries
   (a1) a ligand X¹ selected from F, Cl, Br, I, phosphate and sulfonate, and
   (a2) an organyl ligand R¹ containing at least one non-aromatic carbon-carbon double bond, wherein a coordinative bond between the zinc atom and the ligand R¹ is formed in allylic position relative to the non-aromatic carbon-carbon double bond of the ligand R¹;
(b) Mg(R²)₂, wherein the groups R² are independently selected from carboxylic acid anions; and
(c) LiX², wherein X² is selected from Cl, Br and I.

In accordance with a further aspect, the invention provides a process for the preparation of the organozinc composition in accordance with the invention. In particular, a process is provided for the preparation of an organozinc composition, said process comprising:
reacting (i) elemental zinc with (ii) an organic starting compound containing at least one non-aromatic double bond and a substituent X¹ selected from F, Cl, Br, I, phosphate and sulfonate, which substituent X¹ is bound to a carbon atom in allylic position relative to the non-aromatic carbon-carbon double bond,
to yield an organozinc complex wherein a zinc atom carries a ligand X¹ corresponding to said substituent X¹ and an organyl ligand R¹ containing at least one non-aromatic carbon-carbon double bond, and a coordinative bond between the zinc atom and R¹ is formed in allylic position relative to the non-aromatic carbon-carbon double bond;
wherein the reaction of the elemental zinc and the organic starting compound is carried out in the presence of LiX², wherein X² is selected from Cl, Br and I; and wherein Mg(R²)₂ is provided in the reaction system either prior to, during or after the reaction of the elemental zinc with the organic starting compound, with the groups R² being independently selected from carboxylic acid anions.

In further aspects to be discussed in more detail below, the invention provides synthetic procedures using the organozinc composition in accordance with the invention, i.e. procedures involving the use of the organozinc composition in chemical synthesis as a reagent for the formation of a carbon-carbon bond.

In this context, processes of particular relevance are:
- a process for the preparation of an alcohol compound, said process comprising the step of reacting (i) an aldehyde or a ketone with (ii) an organozinc composition in accordance with the invention;
- a process for the preparation of a β,γ-unsaturated ketone, said process comprising the step of reacting (i) an activated carboxylic acid with (ii) an organozinc composition in accordance with the invention;
- a process for coupling two organic compounds via formation of a carbon-carbon bond, said process comprising the step of reacting
   (i) an organic compound containing at least one non-aromatic double bond and a substituent X³ bound to a carbon atom in allyl-position to the non-aromatic carbon-carbon double bond, wherein X³ is an electrophilic leaving group, and
   (ii) an organozinc composition in accordance with the invention; and
- a process for the preparation of a compound comprising an aryl or heteroaryl moiety, which aryl or heteroaryl moiety carries a substituent containing a non-aromatic carbon-carbon double bond, said process comprising the step of carrying out a cross-coupling reaction between
   (i) a compound comprising an aryl or heteroaryl moiety, which aryl or heteroaryl moiety carries an electrophilic substituent, and
   (ii) an organozinc composition in accordance with the invention,

in the presence of an organometallic compound acting as a catalyst for the cross-coupling reaction.

### Qrganozinc composition

As noted above, the present composition provides an organozinc composition comprising (a) an organozinc complex, (b) a magnesium component Mg(R²)₂, and (c) a lithium component LiX². With a view to its function in organic synthesis, and to its capability of introducing an allyl moiety into another organic compound, the organozinc composition in accordance with the invention may also be referred to as allylzinc reagent herein.

It has been found by the present inventors that the combination of the components (b) and (c) with the organozinc complex (a), which forms the actual active species in the organozinc composition, is necessary to provide the advantageous characteristics of the organozinc compositions as a stable reactant. Without wishing to be bound by theory, it is believed that it is the role of the magnesium component (b) to stabilize the complex (a) in the final organozinc composition, and the role of the lithium component (c) to activate the reagents needed for the synthesis of the organozinc composition, in particular the elemental Zn, such that the composition can be made accessible. It will be understood in this respect that the reference to a composition comprising components (a), (b) and (c) encompasses mixtures between the components, and also encompasses the case that two of the components (a), (b) and (c) or all three of them are further associated e.g. by a coordinative bond between them.

In the organozinc composition in accordance with the invention, the relative amounts of the component (a), component (b) and component (c), expressed as the molar ratio of complexed Zn atoms to Mg atoms and Li atoms, Zn:Mg:Li, are preferably in the range of 1.0:(0.5-2.0):(0.5-2.0). More preferably, the ratio is 1.0:(0.8-1.5):(0.8-1.5), and most preferably 1.0:(1.0-1.2):(1.0-1.2).

Generally and preferably, the organozinc composition in accordance with the invention is a solid composition. As will be understood by the skilled reader, this means in particular that the composition is solid at room temperature (typically 25 °C). As will also be understood, the pressure does not play an important role in this respect, but for reference purposes conditions of e.g. 25 °C and 100 kPa may be indicated. The possibility to provide the organozinc composition in accordance with the invention as a solid composition has been found to be particularly advantageous for its storage stability. While previously known organozinc halides, obtained as a solution e.g. in THF lose a significant part of their activity within a period of several hours, the organozinc compositions in accordance with the invention may be stored under an inert gas, such as argon, for weeks or even months.

### Component (a)

Component (a) of the organozinc composition in accordance with the invention is an organozinc complex, wherein a zinc atom carries (a1) a ligand X¹ selected from F, Cl, Br, I, phosphate and sulfonate, and (a2) an organyl ligand R¹ containing at least one non-aromatic carbon-carbon double bond, wherein a coordinative bond between the zinc atom and the ligand R¹ is formed in allylic position relative to the non-aromatic carbon-carbon double bond of the ligand R¹.

In accordance with this definition, the zinc atom in the organozinc complex (a) is coordinated by at least two ligands, X¹ and R¹. As will be understood by the skilled reader, this encompasses the possibility that further coordinative bonds are formed to the zinc atom to lead to a higher coordination number, e.g. if a halogen atom X¹ from a second Zn complex acts as a bridging ligand which is coordinated to two Zn atoms.

The ligand X¹ is selected from F, Cl, Br, I, phosphate and sulfonate. Preferably, X¹ is selected from Cl, Br, and phosphate, and more preferably from Cl and Br.

As used herein, the term "phosphate" refers to a group or a ligand of the formula -OP(O)(OR¹⁰)₂, wherein the groups R¹⁰ are independently selected from hydrogen, alkyl, cycloalkyl, alkenyl, cycloalkenyl, aralkyl and aryl, preferably from hydrogen, alkyl, and aryl.

As used herein the term "sulfonate" refers to a group or a ligand of the formula -OS(O)₂R¹⁰, wherein the group R¹⁰ is selected from hydrogen, alkyl, cycloalkyl, alkenyl, cycloalkenyl, aralkyl and aryl, preferably from hydrogen, alkyl, and aryl. Optionally, these groups R¹⁰ may be substituted by a halogen substituent, preferably a fluoro substituent. The aryl group or the aryl moiety of the aralkyl group as R¹⁰ may be substituted by a substituent selected from halogen, preferably fluoro, and alkyl, preferably C1-C6 alkyl. Unless indicated otherwise in a specific context, most preferred as sulfonate group is a sulfonate group selected from a tosylate group (i.e. a p-toluenesulfonic acid ester group), a mesylate group (i.e. a methansulfonate group), and a triflate group (i.e. a trifluoromethanesulfonate group).

The organyl ligand R¹ is also referred to herein as an allylic ligand. It contains at least one non-aromatic carbon-carbon double bond, wherein a coordinative bond between the zinc atom and the ligand R¹ is formed in allylic position relative to the non-aromatic carbon-carbon double bond of the ligand R¹. Preferably, the non-aromatic carbon-carbon double bond is further a non-conjugated double bond. As will be understood, the coordinative bond between the zinc atom and the ligand R¹ is generally an σ-bond.

As used herein, and unless otherwise indicated in a specific context, the terms "allyl" or "allylic" refer to a group or a ligand of the formula -CH₂-CH=CH₂ as well as derivatives thereof. Such derivatives may be formed by replacing one or more of the H-atoms by a substituent, including the case that any two suitable substituents are linked to each other such that a group is formed which comprises one or more cyclic moieties, provided that the carbon-carbon double bond indicated in the formula remains non-aromatic, and preferably non-conjugated. In other words, the allyl ligand or group, or the allylic ligand or group is one which contains the following structural moiety: 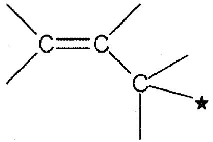 wherein the non-marked open bonds indicate positions where hydrogen atoms or substituents may be attached, including the case that any two suitable substituents are linked to each other such that a group is formed which comprises one or more cyclic moieties, provided that the carbon-carbon double bond indicated in the formula remains non-aromatic, and preferably non-conjugated. The bond marked by the star * indicates the position where the allylic ligand or allylic group is attached to the remainder of the concerned molecule containing the allylic ligand or allylic group.

In line with the above, and with the skilled person's understanding, the reference to a bond in allylic position (or the reference to a bond in allylic position relative to a non-aromatic carbon-carbon double bond) indicates that the respective bond is formed to a sp³-hybridized carbon atom which, in turn, is covalently bound to a sp²-hybridized carbon atom that forms a non-aromatic double bond to another sp²-hybridized carbon atom. Similarly, the reference to an atom, a group or a substituent bound in allylic position (or the reference to an atom, a group or a substituent bound in allylic position relative to a non-aromatic carbon-carbon double bond) indicates that the respective atom, group or substituent is bound to a sp³-hybridized carbon atom which, in turn, is covalently bound to a sp²-hybridized carbon atom that forms a non-aromatic double bond to another sp²-hybridized carbon atom.

It will be understood that the organozinc compositions of the invention provide a versatile tool to make structurally diverse allylic groups accessible in organic synthesis, and the substituents that can be attached to the allylic moiety are not limited. Nevertheless, a number of preferred substituent configurations can be defined.

Thus, preferably, the organyl ligand R¹ is a ligand of formula (I): 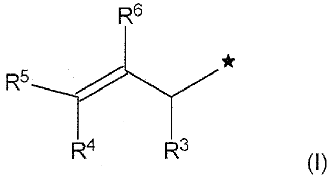 wherein the star * marks the coordinative bond to the zinc atom,
R³ to R⁶ are independently selected from hydrogen, optionally substituted alkyl, optionally substituted cycloalkyl, optionally substituted alkenyl, optionally substituted cycloalkenyl, optionally substituted alkinyl, optionally substituted alkoxy, optionally substituted aryl, optionally substituted heteroaryl, -COOR⁷, -NR⁷R⁸, -SiR⁷R⁸R⁹, F and CN, wherein R⁷ to R⁹ are independently selected from hydrogen, optionally substituted alkyl, optionally substituted cycloalkyl, optionally substituted alkenyl, optionally substituted cycloalkenyl optionally substituted aryl and optionally substituted heteroaryl;
wherein any optionally substituted alkyl, optionally substituted cycloalkyl, optionally substituted alkenyl, optionally substituted cycloalkenyl, optionally substituted alkinyl, and optionally substituted alkoxy may be substituted by one or more substituents independently selected from alkoxy, aryl, -COOR^{7a}, -NR^{7a}R^{8a}, F, and CN, wherein R^{7a} and R^{8a} are independently selected from hydrogen, alkyl, cycloalkyl, alkenyl, cycloalkenyl and aryl;
wherein any optionally substituted aryl and optionally substituted heteroaryl may be substituted by one or more substituents independently selected from alkyl, alkenyl, alkinyl, alkoxy, -COOR^{7a}, -NR^{7a}R^{8a}, F, and CN, wherein R^{7a} and R^{8a} are independently selected from hydrogen, alkyl, cycloalkyl, alkenyl, cycloalkenyl and aryl;
and wherein any two suitable groups selected from R³ to R⁶ may be linked to each other such that a group is formed which comprises one or more cyclic moieties. The carbon-carbon double bond shown in formula (I) will remain non-aromatic in such a cyclic moiety.

As will be understood by the skilled reader, the reference herein to two substituent groups, e.g. groups selected from R³ to R⁶, which are linked to each other such that a group is formed which comprises one or more cyclic moieties typically indicates that a bond is drawn between an atom in the first group and an atom in the second group which can provide a valency to form such a bond, e.g. by formal abstraction of a hydrogen atom. Thus, a cyclic moiety is formed together with the atoms to which the substituent groups are attached. However, it will also be understood that the group formed by linking the substituent groups to each other may contain further structural units apart from the cyclic moiety. For example if two branched substituent groups are linked to each other, a second cyclic moiety or a substituted cyclic moiety may be formed. If two of R³ to R⁶ are linked to each other, the two groups are preferably R³ and R⁴, or R⁵ and R^{6,}, or both.

Preferably, R³ to R⁶ are independently selected from hydrogen, optionally substituted alkyl, optionally substituted cycloalkyl, optionally substituted alkoxy, optionally substituted aryl, optionally substituted heteroaryl, -COOR⁷, -NR⁷R⁸, -SiR⁷R⁸R⁹, F and CN, wherein R⁷ to R⁹ are independently selected from hydrogen, alkyl, cycloalkyl, aryl and heteroaryl;
wherein any optionally substituted alkyl, optionally substituted cycloalkyl, and optionally substituted alkoxy may be substituted by one or more substituents independently selected from alkoxy, aryl, and F;
wherein any optionally substituted aryl and optionally substituted heteroaryl may be substituted by one or more substituents independently selected from alkyl, alkoxy, and F; and wherein any two suitable groups selected from R³ to R⁶ may be linked to each other such that a group is formed which comprises one or more cyclic moieties. As will be understood from the above, the carbon-carbon double bond shown in formula (I) will remain non-aromatic in such a cyclic moiety.

More preferably, R³ to R⁶ are independently selected from hydrogen, alkyl, cycloalkyl, aryl, -COOR⁷, and CN, wherein R⁷ is selected from alkyl, cycloalkyl, and aryl, wherein any two suitable groups selected from R³ to R⁶ may be linked to each other such that a group is formed which comprises one or more cyclic moieties. As will be understood from the above, the carbon-carbon double bond shown in formula (I) will remain non-aromatic in such a cyclic moiety.

For R³ to R⁶ as defined above, including the preferred and more preferred definitions, it is further preferred that two or more, more preferably three or more of R³ to R⁶, and even further preferred all four of R³ to R⁶, are hydrogen or one of the hydrocarbon groups defined above (in particular alkyl or aryl), including the possibility that any two suitable groups selected from R³ to R⁶ may be linked to each other such that a group is formed which comprises one or more cyclic moieties.

Moreover, it is preferred that not more than one of R³ to R⁶ represents a group selected from -COOR⁷, F and CN, wherein R⁷ is defined as above.

For any group -COOR⁷ or -COOR^{7a}, it is preferred that R⁷ or R^{7a}, respectively, are a group other than a hydrogen atom.

In view of the above, examples of particularly preferred constellations for R³ to R⁶ can be listed as follows:
- R³ and R⁶ are hydrogen, and R⁴ and R⁵ are independently selected from hydrogen, alkyl or aryl;
- R³ is hydrogen, R⁴ is selected from hydrogen, alkyl, aryl, -COOR⁷, and CN, wherein R⁷ is selected from hydrogen, alkyl, and aryl, R⁵ and R⁶ are independently selected from hydrogen or alkyl, or are linked to each other to form a cycloalkene moiety with the double bond to which they are attached; or
- R³ and R⁴ are independently selected from hydrogen or alkyl, or are linked to each other to form a cycloalkene with the double bond to which they are attached; and R⁵ and R⁶ are independently selected from hydrogen, alkyl, aryl, -COOR⁷, and CN, wherein R⁷ is selected from alkyl, and aryl, and with the proviso that at least one of R⁵ and R⁶ is selected from hydrogen, alkyl, and aryl.

### Component (b)

Component (b) of the organozinc composition in accordance with the invention is the magnesium component Mg(R²)₂, wherein the groups R² are independently selected from carboxylic acid anions.

A typical structure of the groups R² is thus R¹¹-C(O)O⁻, wherein R¹¹ is selected from alkyl, cycloalkyl and aryl. A preferred alkyl group R¹¹ has a number of carbon atoms of 1 to 10. A preferred cycloalkyl group has a number of carbon atoms of 4 to 10. It will be understood that the term "cycloalkyl" includes bridged cyclic structures. A preferred aryl group is selected from phenyl or naphthyl, and is in particular phenyl. Among alkyl, cycloalkyl and aryl, alkyl and cycloalkyl are preferred.

It is more preferred that at least one, more preferably both groups R² are carboxylic acid anions having a tertiary carbon atom in α-position to the carboxylate group. It will be understood that the tertiary carbon atom in α-position to the carboxylate group is the carbon atom directly bound to the carbon of the carboxylic acid group -C(O)O⁻. In this context, a typical structure of a group R² having a tertiary carbon atom in α-position to the carboxylate group is R^{11a}-C(O)O⁻, wherein R^{11a} is selected from alkyl and cycloalkyl having a tertiary carbon atom directly bound to the carboxylic acid group -C(O)O⁻. Preferred alkyl or cycloalkyl groups R^{11a} have a number of carbon atoms of 4 to 10.

In a more preferred embodiment, the groups R² are selected from a pivalate group (i.e. anions of pivalic acid with the structure tert-butyl-C(O)O⁻) and a 1-admantanecarboxylic acid anion. Most preferably, both groups R² are pivalate groups.

### Component (c)

Component (c) of the organozinc composition in accordance with the invention is the lithium component LiX², wherein X² is selected from Cl, Br and I. Preferably, X² is selected from Cl, and Br, and most preferably X² is Cl.

### Preparation of the organozinc composition

It has been found that the stable organozinc composition in accordance with the present invention can be prepared by a process comprising:
reacting (i) elemental zinc with (ii) an organic starting compound containing at least one non-aromatic carbon-carbon double bond and a substituent X¹ selected from F, Cl, Br, I, phosphate and sulfonate, which substituent X¹ is bound to a carbon atom in allylic position relative to the non-aromatic carbon-carbon double bond,
to yield an organozinc complex wherein a zinc atom carries a ligand X¹ corresponding to said substituent X¹ and an organyl ligand R¹ containing at least one non-aromatic carbon-carbon double bond, and a coordinative bond between the zinc atom and R¹ is formed in allylic position relative to the non-aromatic carbon-carbon double bond;
wherein the reaction of the elemental zinc and the organic starting compound is carried out in the presence of LiX², wherein X² is selected from Cl, Br and I; and
wherein Mg(R²)₂ is provided in the reaction system either prior to, during or after the reaction of the elemental zinc with the organic starting compound, with the groups R² being independently selected from carboxylic acid anions.

As elemental zinc, zinc powder can be conveniently used in the process of the present invention.

The organic starting compound is a compound containing a non-aromatic carbon-carbon double bond as a part of an allylic group as defined in the context of the discussion of component (a). The above explanations, including the definitions of preferred embodiments of the allylic group equally apply for the organic starting compound. Thus, it is preferred that the non-aromatic carbon-carbon double bond is additionally non-conjugated.

The substituent X¹ in the organic starting compound is selected from F, Cl, Br, I, phosphate and sulfonate. Preferably, X¹ is selected from Cl, Br, and phosphate, and more preferably from Cl and Br.

As used herein, the term "phosphate" refers to a group or a ligand of the formula -OP(O)(OR¹⁰)₂, wherein the groups R¹⁰ are independently selected from hydrogen, alkyl, cycloalkyl, alkenyl, cycloalkenyl, aralkyl and aryl, preferably from hydrogen, alkyl, and aryl.

As used herein the term "sulfonate" refers to a group or a ligand of the formula -OS(O)₂R¹⁰, wherein the group R¹⁰ is selected from hydrogen, alkyl, cycloalkyl, alkenyl, cycloalkenyl, aralkyl and aryl, preferably from hydrogen, alkyl, and aryl. Optionally, these groups R¹⁰ may be substituted by a halogen substituent, preferably a fluoro substituent. The aryl group or the aryl moiety of the aralkyl group as R¹⁰ may be substituted by a substituent selected from halogen, preferably fluoro, and alkyl, preferably C1-C6 alkyl. Unless indicated otherwise in a specific context, most preferred as sulfonate group is a sulfonate group selected from a tosyl group (i.e. a p-toluenesulfonic acid ester group), a mesylate group (i.e. a methansulfonate group), and a triflate group (i.e. a trifluoromethanesulfonate group).

As will be understood from the above, during the process for the preparation of the organozinc composition in accordance with the invention, the organozinc complex (a) is formed via an insertion reaction of Zn into the bond which attaches the substituent X¹ to the remainder of the organic starting compound. As a result, the zinc complex is formed with a ligand X¹ corresponding to the substituent X¹ in the former organic starting compound, and the organyl ligand R¹ corresponding to the part of the former organic starting compound to which the substituent X¹ was attached. It will further be understood that the term "corresponding" is used in this context to indicate that the chemical composition and, where applicable, the arrangement of the atoms in the concerned moiety are retained. The electronic structure of the bond will generally undergo certain changes due to the change from a covalent bond between X¹ and the remainder of the molecule in the organic starting compound to coordinative bonds between Zn and X¹ and between Zn and the organyl ligand R¹, respectively, in the organozinc complex. To that extent, the organic starting compound can also be referred to as a compound R¹-X¹, wherein R¹ is an organic group containing at least one non-aromatic carbon-carbon double bond, and a sp3-hybridized carbon atom which is bound directly to one of the carbon atoms linked by the non-aromatic carbon-carbon double bond, and the substituent X¹ is selected from F, Cl, Br, I, phosphate and sulfonate, and is bound to the sp³-hybridized carbon atom which is bound directly to one of the carbon atoms linked by the non-aromatic carbon-carbon double bond.

Preferably, the organic starting compound is a compound of formula (II): 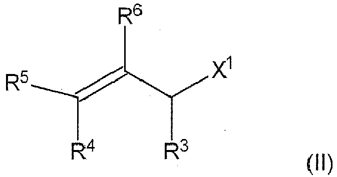 wherein
R³ to R⁶ are independently selected from hydrogen, optionally substituted alkyl, optionally substituted cycloalkyl, optionally substituted alkenyl, optionally substituted cycloalkenyl, optionally substituted alkinyl, optionally substituted alkoxy, optionally substituted aryl, optionally substituted heteroaryl, -COOR⁷, -NR⁷R⁸, -SiR⁷R⁸R⁹, F and CN, wherein R⁷ to R⁹ are independently selected from hydrogen, optionally substituted alkyl, optionally substituted cycloalkyl, optionally substituted alkenyl, optionally substituted cycloalkenyl, optionally substituted aryl and optionally substituted heteroaryl;
wherein any optionally substituted alkyl, optionally substituted cycloalkyl, optionally substituted alkenyl, optionally substituted cycloalkenyl, optionally substituted alkinyl, and optionally substituted alkoxy may be substituted by one or more substituents independently selected from alkoxy, aryl, -COOR^{7a}, -NR^{7a}R^{8a}, F, and CN, wherein R^{7a} and R^{8a} are independently selected from hydrogen, alkyl, cycloalkyl, alkenyl, cycloalkenyl and aryl;
wherein any optionally substituted aryl and optionally substituted heteroaryl may be substituted by one or more substituents independently selected from alkyl, alkenyl, alkinyl, alkoxy, -COOR^{7a}, -NR^{7a}R^{8a}, F, and CN, wherein R^{7a} and R^{8a} are independently selected from hydrogen, alkyl, cycloalkyl, alkenyl, cycloalkenyl and aryl;
and wherein any two suitable groups selected from R³ to R⁶ may be linked to each other such that a group is formed which comprises one or more cyclic moieties. As will be understood from the above, the carbon-carbon double bond shown in formula (I) will remain non-aromatic in such a cyclic moiety;
and wherein X¹ is selected from F, Cl, Br, I, phosphate and sulfonate.

In line with the above, it will be understood that, for the preferred embodiments of R³ to R⁶ and X¹, the explanation provided in the discussion of component (a) above equally applies. Also in this context, it is preferred for any group -COOR⁷ or -COOR^{7a}, that R⁷ or R^{7a}, respectively, are a group other than a hydrogen atom.

As will be understood by the skilled reader, the reference herein to two substituent groups, e.g. groups selected from R³ to R⁶, which are linked to each other such that a group is formed which comprises one or more cyclic moieties typically indicates that a bond is drawn between an atom in the first group and an atom in the second group which can provide a valency to form such a bond, e.g. by formal abstraction of a hydrogen atom. Thus, a cyclic moiety is formed together with the atoms to which the substituent groups are attached. However, it will also be understood that the group formed by linking the substituent groups to each other may contain further structural units apart from the cyclic moiety. For example if two branched substituent groups are linked to each other, a second cyclic moiety or a substituted cyclic moiety may be formed. If two of R³ to R⁶ are linked to each other, the two groups are preferably R³ and R⁴, or R⁵ and R^{6,}, or both.

The reaction between the zinc and the organic starting compound usually takes place in a solvent, preferably in an aprotic solvent, and more preferably in an aprotic polar solvent like THF. Mixtures of solvents can be used. The reaction temperature can be, e.g., between 0°C and the boiling point of the solvent. Preferred are temperatures in the range of 30 to 60 °C.

Preferably, the elemental zinc is used in a molar ratio, relative to the number of moles of the organic starting compound, of 1.0 to 3.0, more preferably 1.5 to 2.5, and most preferred is a molar ratio of 2.0.

An activator for zinc can be added to the reaction system prior to or during the reaction. Examples of activators are 1,2-dibromoethane, iodine, organic and inorganic acids, such as acetic acid, trifluoroacetic acid or hydrochloric acid, and halogenosilanes, such as tetrachlorosilane, trimethylchlorosilane, dimethylchlorosilane or methyltrichlorosilane, as well as combinations thereof. Preferred are 1,2-dibromoethane or iodine, and particularly preferred is 1,2-dibromoethane.

As used herein, the term "reaction system" refers to the vessel wherein the reaction is carried out, and which, unless specifically defined in a given context, may contain one or more of the compounds or solvents which may be involved in the processes of the present invention.

In accordance with the process of the invention, the reaction of the elemental zinc and the organic starting compound is carried out in the presence of LiX². X² is selected from Cl, Br and I. Preferably, X² is selected from Cl, and Br, and most preferably X² is Cl.

Moreover, Mg(R²)₂ is provided in the reaction system either prior to, during or after the reaction of the elemental zinc with the organic starting compound, with the groups R² being independently selected from carboxylic acid anions. For preferred embodiments of R², the explanation provided in the discussion of component (b) above equally applies.

In line with the above, the relative amounts of the organic starting compound providing component (a), the magnesium component and the lithium component used in the process in accordance with the invention, expressed as the molar ratio of organic starting compound to Mg atoms and Li atoms, are preferably in the range of 1.0:(0.5-2.0):(0.5-2.0). More preferably, the ratio is 1.0:(0.8-1.5):(0.8-1.5), and most preferably 1.0:(1.0-1.2):(1.0-1.2).

In order to allow the LiX² to be present during the reaction of the elemental zinc and the organic starting compound, the LiX² can be added as such to the reaction system prior to the reaction of elemental zinc with the organic starting compound. Generally, this means that the addition takes place prior to the addition of one of the two reactants, or prior to both reactants. Conveniently, the LiX² can be added to reaction system before any solvent, which allows the LiX² to be dried via heating.

Alternatively, it is also possible to form LiX² in the reaction system *in-situ,* e.g. by adding Mg(X²)₂ and LiR² to the reaction system prior to or concurrently with the reaction of elemental zinc with the organic starting compound, so that Mg(R²)₂ and LiX² are formed in the reaction system. If this option is chosen, the addition of Mg(X²)₂ and LiR² to the reaction system prior to the reaction of elemental zinc with the organic starting compound is preferred. The definitions of X² and R² are as indicated above.

The Mg(R²)₂ is preferably provided in the reaction system by adding it as such to the reaction system prior to the reaction of elemental zinc with the organic starting compound, and the reaction is carried out in the presence of Mg(R²)₂.

Preferably, the presence of water should be avoided as far as possible in the process for the preparation of an organozinc composition in accordance with the invention. To that extent, it is preferred to adopt one or more of the following measures: carry out the process under protective gas, such as argon or nitrogen; use of dry solvents; and use of dry solid components.

As will be appreciated, it is preferred to remove any solvent from the reaction mixture after the reaction has been carried out so that a solid product is obtained.

### Synthetic procedures using the organozinc composition

As a general aspect, the invention provides the use of the organozinc composition in accordance with the invention in chemical synthesis as a reagent for the formation of a carbon-carbon bond. As a result, the organozinc compositions of the present invention allow allylic groups as defined above to be introduced into another organic compound.

### Reaction with aldehydes or ketones

As a first exemplary type of reaction, the organozinc compositions in accordance with the invention can be used for the preparation of an alcohol compound via reaction with an aldehyde or a ketone.

Thus, the invention further provides a process for the preparation of an alcohol compound, said process comprising the step of reacting (i) an aldehyde or a ketone with (ii) an organozinc composition in accordance with the invention. Preferred as reactants are aldehydes and ketones carrying a C1-substituent, in particular a methyl group, at the carbonyl group.

As a result of the reaction, the organyl ligand R¹ is transferred from the organozinc complex (a) contained in the organozinc composition in accordance with the invention to the aldehyde or the ketone. The former ligand is covalently bound to the carbon atom of the carbonyl group in the aldehyde or the ketone, and the aldehyde or ketone is thus reduced to an alcohol.

It will be understood that the electron structure of the ligand R¹ may be changed during the transfer, such that it will not necessarily be bound to the carbon atom of the carbonyl group with the same atom which had been coordinated to the Zn atom in the organozinc complex (a). Rather, it has been found that the substitutents on the allylic moiety of R¹ determine which of the carbon atoms in the allylic moiety binds to the carbon atom of the carbonyl group of the aldehyde or the ketone.

Advantageously, the reaction proceeds without the need for a catalyst. The resulting alcohol can be provided with excellent diastereoselectivity. Moreover, in cases where the carbon-carbon double bond of the allyl ligand is asymmetrically substituted, the organozinc composition reacts with complete regioselectivity by forming the new carbon-carbon bond from the most substituted end of the allylic moiety.

The reaction can be conveniently carried out in a solvent, preferably in an aprotic solvent, and more preferably in an aprotic polar solvent like THF. Mixtures of solvents can be used. To ensure an advantageous control of the reaction, it is preferably carried out at temperatures < 0°C, such as -78°C. In the reaction, it is preferred to use a slight excess of the organozinc reagent, in terms of the molar ratio of the complexed zinc atoms to the aldehyde or ketone.

For further illustration, the following Table 1 lists reactants and products for the reaction of an aldehyde or a ketone with an organozinc composition in accordance with the invention. For reasons of comprehensibility, the exemplary organozinc composition used in the concerned reaction which comprise an allylzinc complex R-ZnCl, LiCl and Mg(OPiv)₂, are abbreviated as R-ZnOPiv (with R representing the allylic ligand attached to Zn shown in the respective formula). The underlying reactions were carried out over 1 h in THF at -78 °C using 1 eq. of the allylzinc reagent and 0.8 eq. of the carbonyl compound.

**Table 1**

| Entry | Allylzinc Reagent | Carbonyl compound | Product/dr^{a} | Yield^{b} |
|---|---|---|---|---|
| 1 | 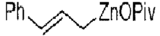 | 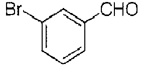 | 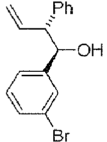 | 86 |
| | | | dr > 99:1 | |
| 2 | 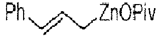 | 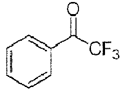 | 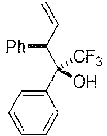 | 71 |
| | | | dr = 92:8 | |
| 3 | 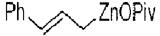 | 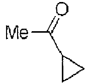 | 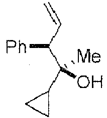 | 96 |
| | | | dr = 94:6 | |
| 4 | 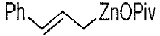 | 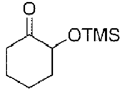 | 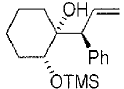 | 94 |
| | | | dr = 89:11 | |
| 5 | 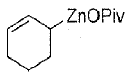 | 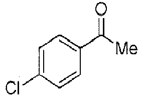 | 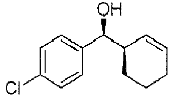 | 92 |
| | | | dr > 99:1 | |
| 6 | 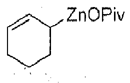 | 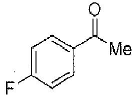 | 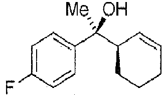 | 89 |
| | | | dr > 99:1 | |
| 7 | 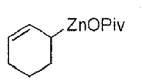 | 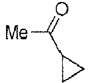 | 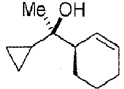 | 92 |
| | | | dr = 94:6 | |
| 8 | 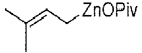 | 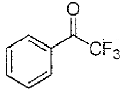 | 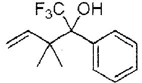 | 87 |
| 9 | 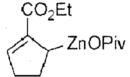 | 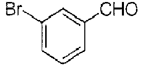 | 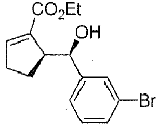 | 91 |
| | | | dr = 97:3 | |

| | | | | |
|---|---|---|---|---|
| a) The diastereoselectivity was determined via ¹H-NMR-analysis (dr = diastereomeric ratio). b) yield in % of isolated, analytically pure product. | | | | |

As underlined by the summary in the table, the products of the reactions using structurally diverse allylzinc reagents are obtained with excellent yield and diastereomeric ratio. Particularly remarkable is the reaction of entry 4, which leads to a product with 3 adjacent stereocenters (dr 89:11)

In addition, the reaction of the allylzinc reactant below with acetylferrocene yields the cyclic product in 92% yield (dr > 99:1) in line with the following reaction scheme. The configuration of the obtained diastereomer was confirmed by X-ray diffraction analysis.

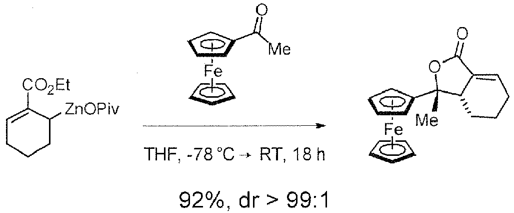

### Reaction with activated carboxylic acids

As a further exemplary type of reaction, the organozinc compositions in accordance with the invention can be used for the preparation of a β,γ-unsaturated ketone, via reaction of (i) an activated carboxylic acid with (ii) an organozinc composition in accordance with the invention.

β,γ-Unsaturated ketones are versatile building blocks in organic chemistry. Although a large number of synthetic methods have been described, only few of them have proven to be practically useful. For example, the acylation of olefins allows the synthesis of β,γ-unsaturated ketones, but also provides α,β-unsaturated ketones as side products. The reaction of different allylic organometal reagents with carboxylic acid chlorides is also disclosed in the literature, but these known methods are difficult to be handled, and thus of little practical value.

Thus, the invention further provides a process for the preparation of a β,γ-unsaturated ketone, said process comprising the step of reacting (i) an activated carboxylic acid with (ii) an organozinc composition in accordance with the invention. Advantageously, also this reaction proceeds without the need for a catalyst.

As used herein, and in accordance with the established meaning in the art, the term β,γ-unsaturated ketone refers to a ketone containing a non-aromatic carbon-carbon double bond between a carbon in β-position and a carbon in γ-position relative to the carbonyl group. In other words, if the carbonyl carbon atom of the β,γ-unsaturated ketone is counted as No. 1, the non-aromatic carbon-carbon double bond is present between carbons No. 3 and 4 in a carbon chain attached to the carbonyl carbon atom.

As a result of the reaction, the organyl ligand R¹ is transferred from the organozinc complex (a) contained in the organozinc composition in accordance with the invention to the activated carboxylic acid. The former ligand is covalently bound to the carbon atom of the carboxyl group in the activated carboxylic acid, and the activated carboxylic acid is thus reduced to a ketone.

It will be understood that the electron structure of the ligand R¹ may be changed during the transfer, such that it will not necessarily be bound to the carbon atom of the carboxyl group with the same atom which had been coordinated to the Zn atom in the organozinc complex (a). Rather, it has been found that the substitutents on the allyl moiety of R¹ determine which of the carbon atoms in the allyl moiety binds to the carbon atom of the carboxyl group.

Suitable types of activated carboxylic acids which can be used in the context of this process are known to the skilled person. Exemplary and preferred types of an activated carboxylic acid are a carboxylic acid chloride, a carboyxlic acid bromide, or a carboxylic acid anhydride. More preferred is a carboxylic acid chloride.

The reaction can be conveniently carried out in a solvent, preferably in an aprotic solvent, and more preferably in an aprotic polar solvent like THF. Mixtures of solvents can be used. To ensure an advantageous control of the reaction, it is preferably carried out at temperatures < 0°C, such as -78°C. In the reaction, it is preferred to use a slight excess of the activated carboxylic acid, in terms of the molar ratio of the complexed zinc atoms to the activated carboxylic acid.

For further illustration, the following Table 2 lists reactants and products for the reaction of carboxylic acid chlorides with an organozinc composition in accordance with the invention. For reasons of comprehensibility, the exemplary organozinc composition used in the concerned reaction which comprise an allylzinc complex R-ZnCl, LiCl and Mg(OPiv)₂ are abbreviated as R-ZnOPiv (with R representing the allylic ligand attached to Zn shown in the respective formula). Unless indicated otherwise, the reactions were carried out over 1 h in THF at -78 °C using 1 eq. of the allylzinc reagent and 1.5 eq. of the activated carboxylic acid.

**Table 2**

| Entry | Allylzinc reagent | Activated carboxlic acid compound | Product | Yield^{a} |
|---|---|---|---|---|
| 1 | 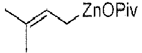 | 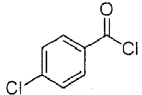 | 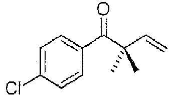 | 93 |
| 2 | 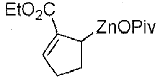 | 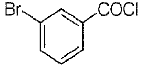 | 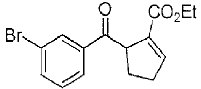 | 68 |
| | | | b,c | |
| 3 | 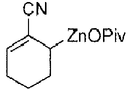 | 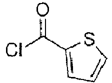 | 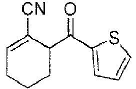 | 77 |
| | | | b,c | |
| 4 | 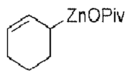 | 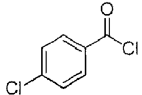 | 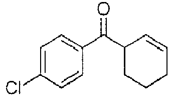 | 78 |

| | | | | |
|---|---|---|---|---|
| a) Yield in % of isolated, analytically pure product; b) the reaction mixture was allowed to warm up to room temperature over night; c) only 0.8 eq. of the carboxylic acid chloride were used. | | | | |

### Allyl-allyl coupling reactions

As yet a further exemplary type of reaction, the organozinc compositions in accordance with the invention can be used for coupling two organic compounds via formation of a carbon-carbon bond via reaction of (i) an organic compound containing at least one non-aromatic double bond and a substituent X³ bound to a carbon atom in allylic-position relative to the non-aromatic carbon-carbon double bond, wherein X³ is an electrophilic leaving group, and (ii) an organozinc composition in accordance with the invention.

Allyl-allyl coupling reactions are of particular importance in organic synthesis since they represent a suitable route for the synthesis of 1,5-dienes.

Thus, the invention further provides a process for coupling two organic compounds via formation of a carbon-carbon bond, said process comprising the step of reacting
(i) an organic compound containing at least one non-aromatic double bond and a substituent X³ bound to a carbon atom in allylic position relative to the non-aromatic carbon-carbon double bond, wherein X³ is an electrophilic leaving group, and
(ii) an organozinc composition in accordance with the invention. Advantageously, also this reaction proceeds without the need for a catalyst.

The organic compound containing the substituent X³ contains an allylic group as defined above, and can thus also be referred to as a compound R¹²-X³, wherein R¹² is an organic group containing at least one non-aromatic carbon-carbon double bond, and a sp³-hybridized carbon atom which is bound directly to one of the carbon atoms linked by the non-aromatic carbon-carbon double bond, and the substituent X³ is bound to the sp3-hybridized carbon atom which is bound directly to one of the carbon atoms linked by the non-aromatic carbon-carbon double bond.

The substituent X³ is an electrophilic leaving group. As will be understood, the term "leaving group" refers in this respect to a group (i.e. a single atom or a chemical moiety formed by a plurality of atoms) which is replaced during the reaction with the organozinc composition in accordance with the invention. Examples include F, Cl, Br, I, phosphate and sulfonate, preferred is Cl, Br or phosphate, and more preferred is Br. For the phosphate and the sulfonate group, the definitions and preferred definitions provided above apply.

As a result of the reaction, the organyl ligand R¹ is transferred from the organozinc complex (a) contained in the organozinc composition in accordance with the invention and replaces the substituent X³ in the organic compound containing this substituent. The former ligand is covalently bound to the organic compound at the position taken by X³ prior to the reaction.

It will be understood that the electron structure of the ligand R¹ may be changed during the transfer, such that it will not necessarily be bound to the organic compound with the same atom which had been coordinated to the Zn atom in the organozinc complex (a). Rather, it has been found that the substituents on the allylic moiety of R¹ determine which of the carbon atoms in the allylic moiety binds to the organic compound acting as a reaction partner.

The reaction can be conveniently carried out in a solvent, preferably in an aprotic solvent, and more preferably in an aprotic polar solvent like THF. Mixtures of solvents can be used. To ensure an advantageous control of the reaction, it is preferably carried out at temperatures below room temperature, e.g. -78 °C to 0 °C.

For further illustration, the following reaction schemes list reactants and products for the allyl-allyl coupling reaction with an organozinc composition in accordance with the invention. For reasons of comprehensibility, the exemplary organozinc composition used in the concerned reaction which comprise an allylzinc complex R-ZnCl, LiCl and Mg(OPiv)₂ are abbreviated as R-ZnOPiv (with R representing the allylic ligand attached to Zn shown in the respective formula).

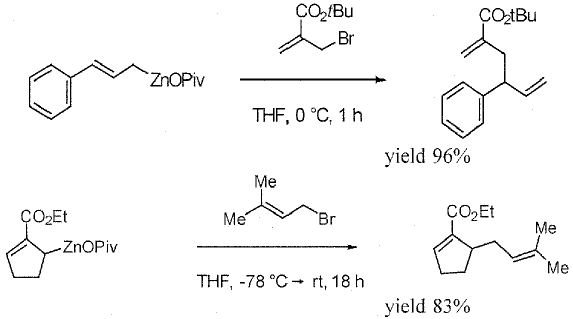

### Cross-coupling reactions

As still a further exemplary type of reaction, the organozinc compositions in accordance with the invention can be used for the preparation of a compound comprising an aryl or heteroaryl moiety, which aryl or heteroaryl moiety carries a substituent containing a non-aromatic carbon-carbon double bond, by carrying out a cross-coupling reaction between
(i) a compound comprising an aryl or heteroaryl moiety, which aryl or heteroaryl moiety carries an electrophilic substituent, and
(ii) an organozinc composition in accordance with the invention,

in the presence of an organometallic compound acting as a catalyst for the cross-coupling reaction.

A significant challenge in the chemistry of allyl-metal-compounds is the cross-coupling with functionalized aromatic compounds, since this reaction frequently proceeds with strictly limited regioselectivity if a non-symmetric allylic nucleophile is used. Remarkably, the organozinc compositions of the present invention allow cross-coupling reactions to be carried out under mild conditions and with excellent selectivity.

Thus, the invention further provides a process for the preparation of a compound comprising an aryl or heteroaryl moiety, which aryl or heteroaryl moiety carries a substituent containing a non-aromatic carbon-carbon double bond, said process comprising the step of carrying out a cross-coupling reaction between
(i) a compound comprising an aryl or heteroaryl moiety, which aryl or heteroaryl moiety carries an electrophilic substituent (which electrophilic substituent is also referred to herein as X⁴), and
(ii) an organozinc composition in accordance with the invention,

in the presence of an organometallic compound acting as a catalyst for the cross-coupling reaction.

As will be understood, the electrophilic substituent is a substituent (i.e. a single atom or a chemical moiety formed by a plurality of atoms) which is directly bound with a covalent bond to a ring atom of the aryl or heteroaryl moiety and which is replaced during the reaction with the organozinc composition in accordance with the invention. Examples include Cl, Br, a tosylate group (i.e. a p-toluenesulfonic acid ester group), a mesylate group (i.e. a methansulfonate group), and a triflate group (i.e. a trifluoromethanesulfonate group). Preferred are Br or a triflate group, and particularly preferred is Br.

As a result of the reaction, the organyl ligand R¹ is transferred from the organozinc complex (a) contained in the organozinc composition in accordance with the invention to the aryl or heteroaryl moiety, where it replaces the electrophilic substituent. The former ligand is thus directly bound with a covalent bond to a ring atom of the aryl or heteroaryl moiety. The product resulting from the reaction is a compound containing an aryl or heteroaryl moiety, which aryl or heteroaryl moiety carries an allylic group as defined above as a substituent, i.e. a substituent wherein a sp2 hybridized carbon atom contained in a carbon-carbon double bond is linked via an sp3 hybridized carbon atom to the aryl or heteroaryl ring.

It will be understood that the electron structure of the ligand R¹ may be changed during the transfer, such that it will not necessarily be bound to the aryl or heteroaryl moiety with the same atom which had been coordinated to the Zn atom in the organozinc complex (a). However, in the cross-coupling reaction in accordance with the present invention, a very high α/γ-selectivity was observed (with the α-position indicating the carbon atom in the ligand R¹ which is coordinated to the Zn atom in the organozinc complex (a) contained in the organozinc composition in accordance with the invention). Independent of the substitution pattern of the ligand R¹, the process in accordance with the invention favors the formation of the α -product.

The cross-coupling reaction is a coupling reaction wherein a carbon-carbon bond is formed via homogeneous catalysis by a transition metal compound. As examples, a Pd-, Pt-, Ni-, or Cu-compound can be mentioned. Preferred is a Pd- or Ni-compound, and in particular a Pd-compound. Typically, the transition metal compound which is used as a catalyst or which is provided in the reaction system to form a catalytically active species *in situ* is an organometallic compound, in particular an organometallic compound containing one of the exemplary and preferred metals mentioned above. An advantage of the organozinc composition in accordance with the invention is that it is able to reacts in a cross-coupling reaction in the presence of a conventional, commercially available catalyst or pre-catalyst, typically under mild reaction conditions. For example, the cross-coupling reaction can be catalyzed by the commercially available PEPPSI™-iPent palladium catalyst (dichloro[1,3-bis(2,6-di-3-pentylphenyl)imidazol-2-ylidene](3-chloropyridyl)palladium(II); cf. Organ et al., Chem. Eur. J. 2006, 12, 4743).

The reaction can be conveniently carried out in a solvent, preferably in an aprotic solvent, and more preferably in an aprotic polar solvent like THF. Mixtures of solvents can be used. The reaction temperature can be, e.g., between 0°C and the boiling point of the solvent. Preferred are temperatures in the range of 30 to 60 °C.

For further illustration, the following Table 3 lists reactants and products for the cross-coupling reaction with an organozinc composition in accordance with the invention. For reasons of comprehensibility, the exemplary organozinc composition used in the concerned reaction which comprise an allylzinc complex R-ZnCl, LiCl and Mg(OPiv)₂ are abbreviated as R-ZnOPiv (with R representing the allyl ligand attached to Zn shown in the respective formula). The reactions were carried out in THF using a PEPPSI™-iPent palladium catalyst (4 mol%) at a temperature of 50 °C. The reaction time was 4 h. It can be seen that structurally diverse aryl or heteroaryl compound can be reacted in the process in accordance with the invention to obtain products with an excellent α/y-selectivity. In each case, at most minimal amounts of the γ-product were obtained.

**Table 3**

| Entry | Allylzinc reagent | Aromatic compound | Product | Yield^{a} |
|---|---|---|---|---|
| 1 | 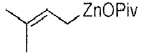 | 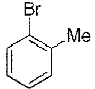 | 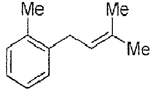 | 70 |
| 2 | 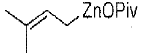 | 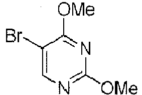 | 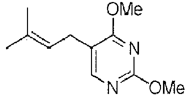 | 77 |
| 3 | 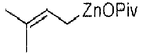 | 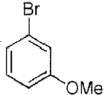 | 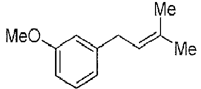 | 71 |
| 4 | 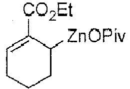 | 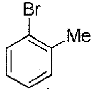 | 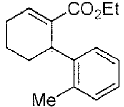 | 79 |
| 5 | 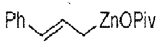 | 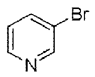 | 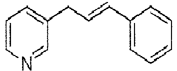 | 69 |
| 6 | 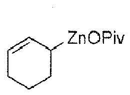 | 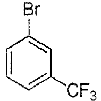 | 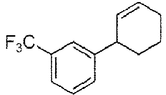 | 72 |

| | | | | |
|---|---|---|---|---|
| a)Yield in % of isolated, analytically pure product. | | | | |

### General definitions of chemical groups

As used herein, "alkyl" represents a straight or branched chain saturated hydrocarbon residue which does not comprise any carbon-carbon (carbon-carbon) double bonds or carbon-carbon triple bonds. As exemplary groups, methyl, ethyl, propyl and butyl are mentioned. Unless specifically defined in a particular context, preferred are C1 to C12 groups, more preferred C1 to C6 groups.

As used herein, "alkenyl" represents a straight or branched chain unsaturated hydrocarbon residue comprising one or more than one (such as two or three) carbon-carbon double bond(s) which does not comprise any carbon-carbon triple bonds. Unless specifically defined in a particular context, preferred are C2 to C12 groups, more preferred C2 to C6 groups.

As used herein, "alkinyl" represents a straight or branched chain unsaturated hydrocarbon residue comprising one or more than one (such as two or three) carbon-to-carbon triple bond(s), and may additionally comprise one or more than one, such as two or three, carbon-carbon double bonds. Unless specifically defined in a particular context, preferred are C2 to C12 groups, more preferred C2 to C6 groups.

As used herein, "aryl" represents an aromatic hydrocarbon ring or ring system, in particular a 6 to 10 membered ring or ring system (unless a different number of ring members is indicated in a specific context), including bridged ring or fused ring systems containing at least one aromatic ring. Preferred as aryl groups are monocyclic groups with 6 ring members or fused bicyclic ring systems with 9 or 10 ring members. Thus, generally preferred embodiments of "aryl" are phenyl or naphthyl. As used herein, and also in the following, a "ring system" refers to a combination of rings, in particular a system of fused rings, a system of spiro rings or a system of rings which are combined in a bridged ring.

As used herein, "aralkyl" represents an alkyl group as defined above, wherein an aryl group as defined above substitutes one of its hydrogen atoms.

As used herein, "heteroaryl" represents an aromatic ring or ring system, preferably a 5-14 membered ring or ring system (unless a different number of ring members is indicated in a specific context), including bridged ring or fused ring systems containing at least one aromatic ring, comprising one or more (such as, e.g., one, two, or three) ring heteroatoms independently selected from O, S, and N. Particularly preferred as heteroaryl groups are monocyclic groups with 5 or 6 ring members and fused bicyclic ring systems with 8 to 10 ring members. "Heteroaryl" may, for example, refer to thienyl; (thiophenyl), benzo[b]thienyl, naphtho[2,3-b]thienyl, thianthrenyl, furyl (furanyl), isobenzofuranyl, chromenyl, xanthenyl, phenoxathiinyl, pyrrolyl (including, without limitation, 2H-pyrrolyl), imidazolyl, pyrazolyl, pyridyl (pyridinyl; including, without limitation, 2-pyridyl, 3-pyridyl, and 4-pyridyl), pyrazinyl, pyrimidinyl, pyridazinyl, indolizinyl, isoindolyl, indolyl (including, without limitation, 3H-indolyl), indazolyl, purinyl, isoquinolyl, quinolyl, phthalazinyl, naphthyridinyl, quinoxalinyl, cinnolinyl, pteridinyl, carbazolyl, beta-carbolinyl, phenanthridinyl, acridinyl, phenanthrolinyl (including, without limitation, [1,10]phenanthrolinyl, [1,7]phenanthro-linyl, and [4,7]phenanthrolinyl), phenazinyl, isothiazolyl, phenothiazinyl, oxazolyl, isoxazolyl, furazanyl, phenoxazinyl, pyrazolo[1,5-a]pyrimidinyl (including, without limitation, pyrazolo[1,5-a]pyrimidin-3-yl), 1,2-benzoisoxazol-3-yl, or benzimidazolyl.

As used herein, "cycloalkyl" or a "cycloalkane" represents a saturated hydrocarbon ring or ring system, preferably a 3-11 membered ring or ring system (unless a different number of ring members is indicated in a specific context), including bridged ring, spiro ring or fused ring systems. "Cycloalkyl" may, for example, refer to cyclopropyl, cyclobutyl, cyclopentyl, cyclohexyl, or cycloheptyl. Preferred as cycloalkyl groups are monocyclic groups with 5 or 6 ring members or fused bicyclic ring systems with 9 or 10 ring members.

As used herein, "cycloalkenyl" or a "cycloalkene" represents a hydrocarbon ring or ring system, preferably a 3-11 membered ring or ring system (unless a different number of ring members is indicated in a specific context), including bridged ring, spiro ring or fused ring systems which contains one or more, preferably one carbon-carbon double bond. "Cycloalkenyl" or "cycloalkene" may, for example, refer to cyclobutyl/cyclobutene, cyclopentyl/cyclopentene or cyclohexyl/cyclohexene. Preferred are monocyclic rings with 5 or 6 ring members and containing one carbon-carbon double bond.

As used herein, "alkoxy" refers to a group -O-alkyl, with alkyl being defined as above, including the preferred meanings.

Important aspects of the invention are summarized in the following:
1. An organozinc composition comprising:
   (a) an organozinc complex, wherein a zinc atom carries
      (a1) a ligand X¹ selected from F, Cl, Br, I, phosphate and sulfonate, and (a2) an organyl ligand R¹ containing at least one non-aromatic carbon-carbon double bond, wherein a coordinative bond between the zinc atom and the ligand R¹ is formed in allylic position relative to the non-aromatic carbon-carbon double bond of the ligand R¹;
   (b) Mg(R²)₂, wherein the groups R² are independently selected from carboxylic acid anions; and
   (c) LiX², wherein X² is selected from Cl, Br and I.
2. The organozinc composition of item 1 which is a solid composition, in particular a solid composition which is solid at a temperature of 25 °C.
3. The organozinc composition of item 1 or 2, wherein the ligand X¹ is selected from Cl and Br.
4. The organozinc composition of any of items 1 to 3, wherein both groups R² are carboxylic acid anions having a tertiary carbon atom in α-position to the carboxylate group.
5. The organozinc composition any of items 1 to 3, wherein both groups R² are pivalate groups.
6. The organozinc composition of any of items 1 to 5, wherein the ligand R¹ is a ligand of formula (I): 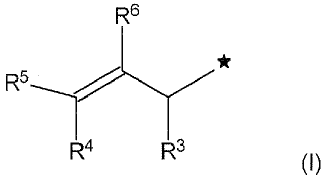 wherein the star * marks the coordinative bond to the zinc atom,
   R³ to R⁶ are independently selected from hydrogen, optionally substituted alkyl, optionally substituted cycloalkyl, optionally substituted alkenyl, optionally substituted cycloalkenyl, optionally substituted alkinyl, optionally substituted alkoxy, optionally substituted aryl, optionally substituted heteroaryl, -COOR⁷, -NR⁷R⁸, -SiR⁷R⁸R⁹, F and CN, wherein R⁷ to R⁹ are independently selected from hydrogen, optionally substituted alkyl, optionally substituted cycloalkyl, optionally substituted alkenyl, optionally substituted cycloalkenyl, optionally substituted aryl and optionally substituted heteroaryl;
      wherein any optionally substituted alkyl, optionally substituted cycloalkyl, optionally substituted alkenyl, optionally substituted cycloalkenyl, optionally substituted alkinyl, and optionally substituted alkoxy may be substituted by one or more substituents independently selected from alkoxy, aryl, -COOR^{7a}, -NR^{7a}R^{8a}, F, and CN, wherein R^{7a} and R^{8a} are independently selected from hydrogen, alkyl, cycloalkyl, alkenyl, cycloalkenyl and aryl; wherein any optionally substituted aryl and optionally substituted heteroaryl may be substituted by one or more substituents independently selected from alkyl, alkenyl, alkinyl, alkoxy, -COOR^{7a}, -NR^{7a}R^{8a}, F, and CN, wherein R^{7a} and R^{8a} are independently selected from hydrogen, alkyl, cycloalkyl, alkenyl, cycloalkenyl and aryl;
   and wherein any two suitable groups selected from R³ to R⁶ may be linked to each other such that a group is formed which comprises one or more cyclic moieties.
7. The organozinc composition of any or items 1 to 6, wherein X² is Cl.
8. The organozinc compostion of any of items 1 to 7, wherein the relative amounts of the component (a), component (b) and component (c), expressed as the molar ratio of complexed Zn atoms to Mg atoms and Li atoms, are in the range of 1.0:(0.8-1.5):(0.8-1.5).
9. A process for the preparation of an organozinc composition, said process comprising:
   reacting (i) elemental zinc with (ii) an organic starting compound containing at least one non-aromatic carbon-carbon double bond and a substituent X¹ selected from F, Cl, Br, I, phosphate and sulfonate, which substituent X¹ is bound to a carbon atom in allylic position relative to the non-aromatic carbon-carbon double bond,
   to yield an organozinc complex wherein a zinc atom carries a ligand X¹ corresponding to said substituent X¹ and an organyl ligand R¹ containing at least one non-aromatic carbon-carbon double bond, and a coordinative bond between the zinc atom and R¹ is formed in allylic position relative to the non-aromatic carbon-carbon double bond;
   wherein the reaction of the elemental zinc and the organic starting compound is carried out in the presence of LiX², wherein X² is selected from Cl, Br and I; and
   wherein Mg(R²)₂ is provided in the reaction system either prior to, during or after the reaction of the elemental zinc with the organic starting compound, with the groups R² being independently selected from carboxylic acid anions.
10. The process of item 9, wherein the organozinc composition is a solid composition, in particular a solid composition which is solid at a temperature of 25 °C.
11. The process of item 9 or 10, wherein the ligand X¹ is selected from Cl and Br.
12. The process of any of items 9 to 11, wherein both groups R² are carboxylic acid anions having a tertiary carbon atom in α-position to the carboxylate group.
13. The process any of items 9 to 11, wherein both groups R² are pivalate groups.
14. A process for the preparation of the organozinc composition as defined in any of claims 1 to 8, said process comprising:
   reacting (i) elemental zinc with (ii) an organic starting compound containing at least one non-aromatic carbon-carbon double bond and a substituent X¹ selected from F, Cl, Br, I, phosphate and sulfonate, which substituent X¹ is bound to a carbon atom in allylic position relative to the non-aromatic carbon-carbon double bond,
   to yield the organozinc complex (a) wherein a zinc atom carries a ligand X¹ corresponding to said substituent X¹ and an organyl ligand R¹ containing at least one non-aromatic carbon-carbon double bond, and a coordinative bond between the zinc atom and R¹ is formed in allylic position relative to the non-aromatic carbon-carbon double bond;
   wherein the reaction of the elemental zinc and the organic starting compound is carried out in the presence of LiX², wherein X² is selected from Cl, Br and I; and
   wherein Mg(R²)₂ is provided in the reaction system either prior to, during or after the reaction of the elemental zinc with the organic starting compound, with the groups R² being independently selected from carboxylic acid anions.
15. The process of any of items 9 to 14, wherein the LiX² is added to the reaction system prior to the reaction of elemental zinc with the organic starting compound.
16. The process of any of items 9 to 15, wherein the Mg(R²)₂ is provided in the reaction system by adding it to the reaction system prior to the reaction of elemental zinc with the organic starting compound, and the reaction is carried out in the presence of Mg(R²)₂.
17. The process of any of items 9 to 14, wherein the Mg(R²)₂ is provided by adding Mg(X²)₂ and LiR² to the reaction system prior to the reaction of elemental zinc with the organic starting compound, so that Mg(R²)₂ and LiX² are formed in the reaction system in-*situ.*
18. The process of any of items 9 to 17, wherein the organic starting compound is a compound of formula (II): 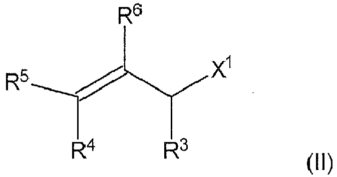 wherein
   R³ to R⁶ are independently selected from hydrogen, optionally substituted alkyl, optionally substituted cycloalkyl, optionally substituted alkenyl, optionally substituted cycloalkenyl, optionally substituted alkinyl, optionally substituted alkoxy, optionally substituted aryl, optionally substituted heteroaryl, -COOR⁷, -NR⁷R⁸, -SiR⁷R⁸R⁹, F and CN, wherein R⁷ to R⁹ are independently selected from hydrogen, optionally substituted alkyl, optionally substituted cycloalkyl, optionally substituted alkenyl, optionally substituted cycloalkenyl, optionally substituted aryl, and optionally substituted heteroaryl,
   wherein any optionally substituted alkyl, optionally substituted cycloalkyl, optionally substituted alkenyl, optionally substituted cycloalkenyl, optionally substituted alkinyl, and optionally substituted alkoxy may be substituted by one or more substituents independently selected from alkoxy, aryl, -COOR^{7a}, -NR^{7a}R^{8a}, F, and CN, wherein R^{7a} and R^{8a} are independently selected from hydrogen, alkyl, cycloalkyl, alkenyl, cycloalkenyl and aryl,
   wherein any optionally substituted aryl and optionally substituted heteroaryl may be substituted by one or more substituents independently selected from alkyl, alkenyl, alkinyl, alkoxy, -COOR^{7a}, -NR^{7a}R^{8a}, F, and CN, wherein R^{7a} and R^{8a} are independently selected from hydrogen, alkyl, cycloalkyl, alkenyl, cycloalkenyl and aryl;
   and wherein any two suitable groups selected from R³ to R⁶ may be linked to each other such that a group is formed which comprises one or more cyclic moieties;
   and wherein X¹ is selected from F, Cl, Br, I, phosphate and sulfonate.
19. Use of the organozinc composition of any of items 1 to 8 or the organozinc composition obtainable by the process of any of items 9 to 18 in chemical synthesis as a reagent for the formation of a carbon-carbon bond.
20. A process for the preparation of an alcohol compound, said process comprising the step of reacting (i) an aldehyde or a ketone with (ii) an organozinc composition of any of items 1 to 8 or the organozinc composition obtainable by the process of any of items 9 to 18.
21. A process for the preparation of a β,γ-unsaturated ketone, said process comprising the step of reacting (i) an activated carboxylic acid with (ii) an organozinc composition of any of items 1 to 8 or the organozinc composition obtainable by the process of any of items 9 to 18.
22. The process of item 21, wherein the activated carboxylic acid is selected from a carboxylic acid chloride and a carboxylic acid anhydride.
23. A process for coupling two organic compounds via formation of a carbon-carbon bond, said process comprising the step of reacting
   (i) an organic compound containing at least one non-aromatic double bond and a substituent X³ bound to a carbon atom in allyl-position to the non-aromatic carbon-carbon double bond, wherein X³ is an electrophilic leaving group, and
   (ii) an organozinc composition of any of items 1 to 8 or the organozinc composition obtainable by the process of any of items 9 to 18.
24. The process of any of items 20 to 23, which is carried out in the absence of a catalyst.
25. A process for the preparation of a compound comprising an aryl or heteroaryl moiety, which aryl or heteroaryl moiety carries a substituent containing a non-aromatic carbon-carbon double bond, said process comprising the step of carrying out a cross-coupling reaction between
   (i) a compound comprising an aryl or heteroaryl moiety, which aryl or heteroaryl moiety carries an electrophilic substituent, and
   (ii) an organozinc composition of any of items 1 to 8 or the organozinc composition obtainable by the process of any of items 9 to 18,

in the presence of an organometallic compound acting as a catalyst for the cross-coupling reaction.

### Examples

### 1. General Information

### General Methodologies

All reactions were carried out under an argon atmosphere in flame-dried glassware. Syringes which were used to transfer anhydrous solvents or reagents were purged with argon prior to use. THF was continuously refluxed and freshly distilled from sodium benzophenone ketyl under nitrogen and stored over molecular sieves. AcOEt was purchased from Sigma-Aldrich with a purity of 99 % and used without distillation or drying prior to use. Yields refer to isolated yields of compounds estimated to be >95% pure as determined by ¹H-NMR (25 °C) and capillary GC. Column chromatography was performed using SiO₂ (0.040-0.063 mm, 230-400 mesh ASTM) from Merck.

Melting points were measured using a Büchi B-540 apparatus and are uncorrected. NMR spectra were recorded in CDCl₃ or C₆D₆ and chemical shifts are reported in parts per million (ppm). Abbreviations for signal coupling are as follows: s, singlet; d, doublet; t, triplet; q, quartet; m, multiplet; br, broad. Mass spectra and high resolution mass spectra (HRMS) were recorded using electron ionization (EI) except otherwise noted. GCs were recorded on machines of the types Hewlett-Packard 6890 or 5890 Series II (Hewlett Packard, 5 % phenylmethylpolysiloxane; length: 10 m, diameter: 0.25 mm; film thickness: 0.25 µm). All reagents not listed in the supporting information were obtained from commercial sources. Liquid aldehydes and acid chlorides were distilled prior to use. Zinc dust (> 98%) was obtained from Sigma Aldrich. MgBu₂ was purchased from Rockwood Lithium. Compounds **2c** (P. E. Peterson, G. Grant, *J. Org. Chem.* **1991,** 56, 16) **2e** (Z. Peng, T. D. Blümke, P. Mayer, P. Knochel, *Angew. Chem. Int. Ed.* **2010,** 49, 8516), **2f, 2g** (C. Sämann, P. Knochel, *Synthesis* **2013,** 1870), **2h** (J.-B. Langlois, A. Alexakis, *Adv. Synth. Catal.* **2010,** 352, 447), and **5g** (Y. Y. Novikov, P. Sampson, Org. Lett. 2003, 5, 2263) were prepared according to literature known procedures.

### Titration of Organozinc Reagents Using Iodine

According to the procedure described in A. Krasovskiy, P. Knochel, Synthesis 2006, 890, accurately weighted aliquots (350 mg) of the crude organozinc material were dissolved in dry THF, so that the total volume of the solution was 2 mL. To the solution thus formed, a standard solution of iodine (1 M, in dry THF) was added until the complete appearance of the dark brown colour of iodine. Thus, the concentration of the active species (in mmol/g) is determined and on this basis the yield of the insertion.

### Stability Studies of Allylzinc Reagents under Argon

To evaluate the stability of organozinc reagents under argon, accurately weighed aliquots of the solid material were placed in Schlenk-flasks at 25 °C. After an extended period of time under Argon at the given temperature the solid material was dissolved in THF and the resulting solution was titrated against iodine, according to the procedure described above and the measured concentration was compared to the one before the storage. The stability of the allylzinc reagents is presented in Table 4.

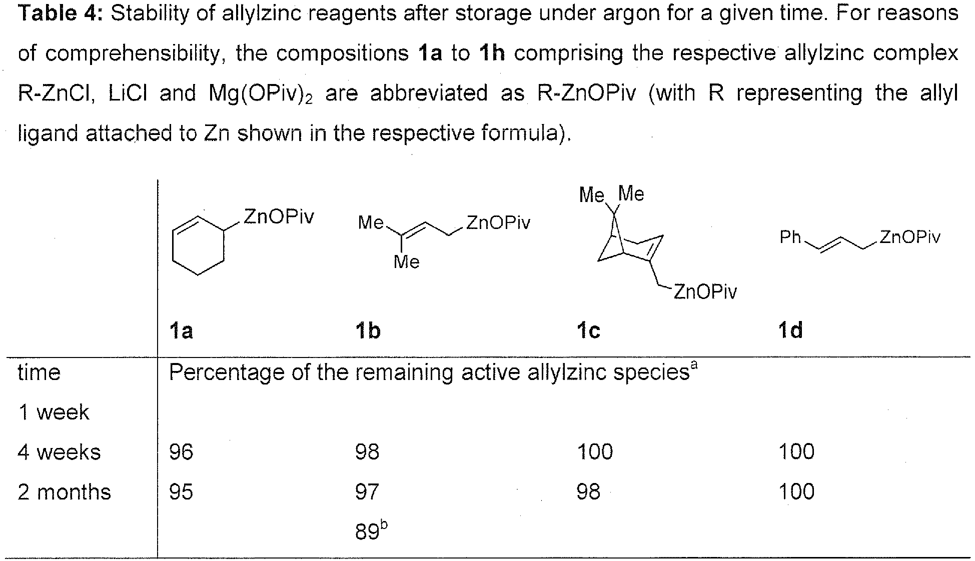

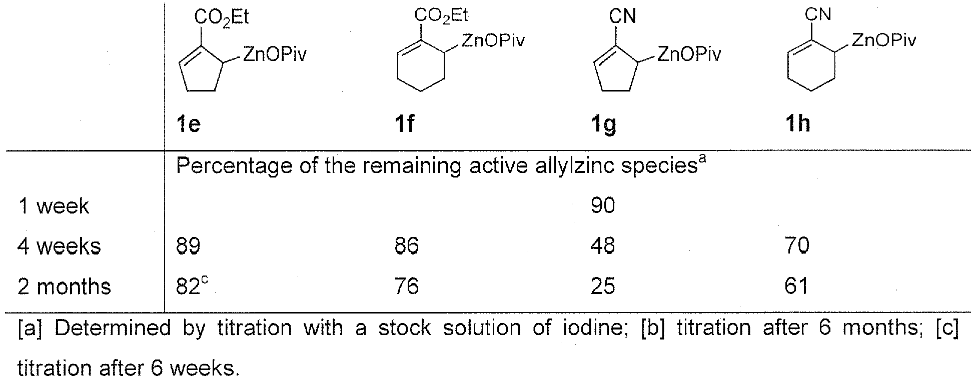

### Preparation of Mg(OPiv)₂

Pivalic acid (20.4 g, 22.6 mL, 200 mmol) was placed in a dry and argon-flushed 500 mL Schlenk-flask, equipped with a magnetic stirring bar and a septum, and dissolved in dry THF (100 mL). The solution was cooled to 0 °C and dibutylmagnesium (148 mL, 1.49 M in hexane, 220 mmol) was added over a period of 30 min under vigorous stirring. Then, the icebath was removed and stirring continued at 25 °C for 6 additional hours at which point bubbling was ceased (a white precipitation was formed). The solvent was removed *in vacuo* and the solid residue was dried for at least four additional hours. Mg(OPiv)₂ was received in quantitative yield, as a puffy amorphous white solid.

### 2. Preparation and Reactions of the Allylzinc Reagents

For reasons of comprehensibility, the allylzinc reagents, i.e. the organozinc composition comprising the organozinc complex R-ZnCl, LiCl and Mg(OPiv)₂ prepared in the examples section, are abbreviated as R-ZnOPiv (with R representing the allylic ligand attached to the Zn atom expressed in the respective formula) and referred to as allylzinc pivalates (with the specific allylic group being indicated). By indicating the presence of the R² group (in the examples, a pivalate was used), the difference of the organozinc composition in accordance with the invention on the one hand, which contain the component Mg(R²)₂, and simple organozinc halides on the other hand is underlined. However, as noted above, this is only an abbreviation, and does not indicate that a OPiv ligand needs to be coordinated with the Zn in the respective organozinc compositions. This convention also applies in the examples wherein the organozinc composition in accordance with the invention is used as synthetic intermediate. Molar concentrations of the organozinc composition are indicated in terms of moles of the Zn complex.

### Typical Procedures

### Typical Procedure for the Preparation of the Allylzinc Reagents of Type 1 (TP1):

A dry and argon-flushed Schlenk-flask, equipped with a magnetic stirring bar and a septum, was charged with Mg(OPiv)₂ (2.72 g, 12.0 mmol) and LiCl (466 mg, 11.0 mmol). The mixture was heated to 330 °C for 10 min under high vacuum followed by the addition of zinc dust (1.31 g, 20.0 mmol). The vessel was evacuated and backfilled with argon. THF (10 mL) and 1,2-dibromoethane (215 µL, 2.50 mmol) were added via syringe and the reaction mixture was heated to 50 °C until bubbling occurred. The allylic halide (10.0 mmol) was added as a solution in THF (10 mL) over a period of 30 min using a syringe pump. The mixture was stirred for the given time at room temperature before the zinc powder was allowed to settle down. The supernatant solution was carefully filtrated using a syringe filter and the yield of the resulting solution of the allylzinc reagent (1) (as represented by compounds **1a** to **1h** above) was determined by iodometric titration.

### Typical Procedure for the Cross-Coupling of the Allylzinc Reagents of Type 1 with Aryl Halides of Type 2 (TP2):

In a dry and argon-flushed Schlenk-flask, equipped with a magnetic stirring bar and a septum, the solid organozinc reagent (1.0 equiv) was dissolved in dry THF or the previously solved organozinc solution was added via syringe. PEPPSI-IPent (2 mol%) and the aryl bromide (0.80 equiv) were added and the mixture was stirred 4 h at 50 °C. Then a sat. aq. NH₄Cl solution (5 mL) was added, the aqueous layer was extracted with EtOAc (3 × 10 mL), washed with solutions of NaHCO₃ (5 mL, sat. aq.) and NaCl (5 mL, sat. aq.) and the combined organic phases were dried over MgSO₄. Evaporation of the solvents *in vacuo* and purification by column chromatography afforded the expected products.

### Typical Procedure for the Addition of the Allylzinc Reagents of Type 1 to Aldehydes and Ketones of Type 5 or to Allylhalogenides of Type 8 (TP3):

In a dry and argon-flushed Schlenk-flask, equipped with a magnetic stirring bar and a septum, the solid organozinc reagent (1.0 equiv) was dissolved in dry THF or the previously solved organozinc solution was added via syringe. The solution was cooled to -78 °C and the carbonyl compound was added was added and the mixture was stirred 1 h at -78 °C. Then a sat. aq. NH₄Cl solution (5 mL) was added, the aqueous layer was extracted with EtOAc (3 × 10 mL), washed with solutions of NaHCO₃ (5 mL, sat. aq.) and NaCl (5 mL, sat. aq.) and the combined organic phases were dried over MgSO₄. Evaporation of the solvents *in vacuo* and purification by column chromatography afforded the expected products.

### Typical Procedure for the Reaction of the Allylzinc Reagents of Type 1 to Acid Chlorides of Type 7 (TP4):

In a dry and argon-flushed Schlenk-flask, equipped with a magnetic stirring bar and a septum, the solid organozinc reagent (1.0 equiv) was dissolved in dry THF or the previously solved organozinc solution was added via syringe. The solution was cooled to -78 °C and the acid chloride was added was added and the mixture was stirred 1 h at -78 °C. Then a sat. aq. NH₄Cl solution (5 mL) was added, the aqueous layer was extracted with EtOAc (3 × 10 mL), washed with solutions of NaHCO₃ (5 mL, sat. aq.) and NaCl (5 mL, sat. aq.) and the combined organic phases were dried over MgSO₄. Evaporation of the solvents *in vacuo* and purification by column chromatography afforded the expected products.

### Preparation of the Allylzinc Reagents

### Cyclohex-2-en-1-ylzinc pivalate (1a)

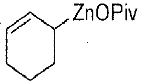

According to **TP1** to a mixture of zinc dust (1.31 g, 20.0 mmol), LiCl (466 mg, 11.0 mmol), Mg(OPiv)₂ (2.72 g, 12.0 mmol) and THF (10 mL) was added 3-bromocyclohex-1-ene (1.61 g, 10 mmol) in 10 mL THF. After 1 h at 25 °C and subsequent evaporation of the solvent Cyclohex-2-en-1-ylzinc pivalate was obtained as a colorless solid. The content of active zinc species was determined by titration with a stock solution of iodine (1.00 M in THF). A concentration of 1.08 mmol/g, which corresponds to a yield of 67% was determined.

### Prenylzinc pivalate (1 b)

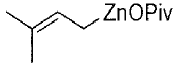

According to **TP1** to a mixture of zinc dust (1.31 g, 20.0 mmol), LiCl (466 mg, 11.0 mmol), Mg(OPiv)₂ (2.72 g, 12.0 mmol) and THF (10 mL) was added prenylbromide (1.49 g, 10 mmol) in 10 mL THF. After 1 h at 25 °C and subsequent evaporation of the solvent prenylzinc pivalate was obtained as a colorless solid. The content of active zinc species was determined by titration with a stock solution of iodine (1.00 M in THF). A concentration of 1.01 mmol/g, which corresponds to a yield of 74% was determined.

### Myrtenylzinc pivalate (1c)

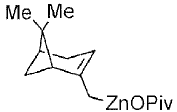

According to **TP1** to a mixture of zinc dust (1.31 g, 20.0 mmol), LiCl (466 mg, 11.0 mmol), Mg(OPiv)₂ (2.72 g, 12.0 mmol) and THF (10 mL) was added myrtenylbromide (2.15 g, 10 mmol) in 10 mL THF. After 1 h at 25 °C and subsequent evaporation of the solvent myrtenylzinc pivalate was obtained as a colorless solid. The content of active zinc species was determined by titration with a stock solution of iodine (1.00 M in THF). A concentration of 0.96 mmol/g, which corresponds to a yield of 77% was determined.

### Cinnamylzinc pivalate (1d)

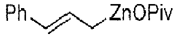

According to **TP1** to a mixture of zinc dust (1.31 g, 20.0 mmol), LiCl (466 mg, 11.0 mmol), Mg(OPiv)₂ (2.72 g, 12.0 mmol) and THF (10 mL) was added cinnamylchloride (1.52 g, 10 mmol) in 10 mL THF. After 1 h at 25 °C and subsequent evaporation of the solvent cinnamylzinc pivalate was obtained as a colorless solid. The content of active zinc species was determined by titration with a stock solution of iodine (1.00 M in THF). A concentration of 0.95 mmol/g, which corresponds to a yield of 78% was determined.

### (2-(Ethoxycarbonyl)cyclopent-2-en-1-yl)zinc pivalate (1e)

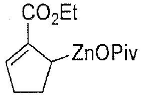

According to **TP1** to a mixture of zinc dust (1.31 g, 20.0 mmol), LiCl (466 mg, 11.0 mmol), Mg(OPiv)₂ (2.72 g, 12.0 mmol) and THF (10 mL) was added ethyl 5-chlorocyclopent-1-enecarboxylate (1.75 g, 10 mmol) in 10 mL THF. After 3 h at 25 °C and subsequent evaporation of the solvent (2-(ethoxycarbonyl)cyclopent-2-en-1-yl)zinc pivalate was obtained as a yellow solid. The content of active zinc species was determined by titration with a stock solution of iodine (1.00 M in THF). A concentration of 0.97 mmol/g, which corresponds to a yield of 51% was determined.

### (2-(Ethoxycarbonyl)cyclohex-2-en-1-yl)zinc pivalate (1f)

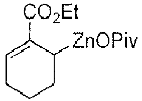

According to **TP1** to a mixture of zinc dust (1.31 g, 20.0 mmol), LiCl (466 mg, 11.0 mmol), Mg(OPiv)₂ (2.72 g, 12.0 mmol) and THF (10 mL) was added ethyl 6-chlorocyclohex-1-enecarboxylate (1.89 g, 10 mmol) in 10 mL THF. After 3 h at 25 °C and subsequent evaporation of the solvent (2-(ethoxycarbonyl)cyclohex-2-en-1-yl)zinc pivalate was obtained as a yellow solid. The content of active zinc species was determined by titration with a stock solution of iodine (1.00 M in THF). A concentration of 1.23 mmol/g, which corresponds to a yield of 90% was determined.

### (2-Cyanocyclopent-2-en-1-yl)zinc pivalate (1g)

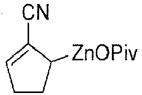

According to **TP1** to a mixture of zinc dust (1.31 g, 20.0 mmol), LiCl (466 mg, 11.0 mmol), Mg(OPiv)₂ (2.72 g, 12.0 mmol) and THF (10 mL) was added 5-bromocyclopent-1-enecarbonitrile (1.72 g, 10 mmol) in 10 mL THF. After 3h at 25 °C and subsequent evaporation of the solvent (2-cyanocyclopent-2-en-1-yl)zinc pivalate was obtained as a dark yellow solid. The content of active zinc species was determined by titration with a stock solution of iodine (1.00 M in THF). A concentration of 1.04 mmol/g, which corresponds to a yield of 81% was determined.

### (2-Cyanocyclohex-2-en-1-yl)zinc pivalate (1e)

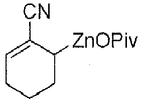

According to **TP1** to a mixture of zinc dust (1.31 g, 20.0 mmol), LiCl (466 mg, 11.0 mmol), Mg(OPiv)₂ (2.72 g, 12.0 mmol) and THF (10 mL) was added 6-chlorocyclohex-1-enecarbonitrile (1.41 g, 10 mmol) in 10 mL THF. After 3 h at 25 °C and subsequent evaporation of the solvent (2-cyanocyclohex-2-en-1-yl)zinc pivalate was obtained as a yellow solid. The content of active zinc species was determined by titration with a stock solution of iodine (1.00 M in THF). A concentration of 1.00 mmol/g, which corresponds to a yield of 55% was determined.

### Preparation of Cross-Coupling Products

### 2,4-Dimethoxy-5-(3-methylbut-2-en-1-yl)pyrimidine (4a)

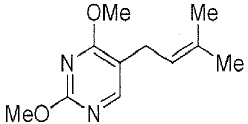

According to **TP2** prenylzinc pivalate **1 b** in dry THF (0.38 M, 3.12 mL, 1.20 mmol) was added to 5-bromo-2,4-dimethoxypyrimidine (219 mg, 1.00 mmol) and PEPPSI-IPent (16 mg, 0.02 mmol) in THF (0.5 mL) and the mixture was stirred for 4 h at 50 °C. Purification of the crude product by flash chromatography (silica gel, isohexane/EtOAc = 10:1) afforded the title compound as a colourless oil (160 mg, 0.77 mmol, 77%).

**¹H-NMR (300 MHz, CDCl₃):** δ / ppm = 5.16 (s, 1 H), 4.36 - 4.26 (m, 1 H), 2.93 (s, 3H), 2.90 (s, 3H), 2.28 (d, J = 7.3 Hz, 2H), 0.71 (s, 3H), 0.64 (s, 3H).

**¹³C**-**NMR (75 MHz, CDCl₃):** δ / ppm = 172.4, 172.1, 165.4, 135.0, 119.6, 99.3, 54.7, 53.8, 36.4, 25.9, 18.1.

**IR (Diamond-ATR, neat):** *ν̃*/cm⁻¹ = 3185, 2982, 2955, 2871, 1584, 1559, 1478, 1457, 1376, 1346, 1282, 1202, 1146, 1097, 1058, 1038, 1007, 9834, 933, 827, 786, 713, 663.

**MS (EI, 70 eV):** *m*/*z* (%) = 208 (M+, 73), 193 (100), 177 (20), 167 (46), 154 (11), 135 (10), 125 (11), 106 (8), 93 (9), 82 (8), 72 (10).

HRMS **(EI):** *m*/*z* calc. for [C₁₁H₁₆N₂O₂]: 208.1212; found: 208.1196 (M⁺).

### Ethyl 2'-methyl-1,4,5,6-tetrahydro-[1,1'-biphenyl]-2-carboxylate (4b)

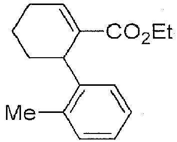

According to **TP2** (2-(ethoxycarbonyl)cyclohex-2-en-1-yl)zinc pivalate **1e** in dry THF (0.40 M, 1.25 mL, 0.50 mmol) was added to 3-bromotoluene (68 mg, 0.40 mmol) and PEPPSI-IPent (8 mg, 0.01 mmol) in THF (0.5 mL) and the mixture was stirred for 4 h at 50 °C. Purification of the crude product by flash chromatography (silica gel, isohexane/EtOAc = 30:1) afforded the title compound as a colourless oil (77 mg, 0.32 mmol, 79%).

**¹H-NMR (300 MHz, CDCl₃):** δ / ppm = 7.26 - 7.21 (m, 1 H), 7.18 - 7.11 (m, 1 H), 7.11 - 7.02 (m, 2H), 7.00 - 6.91 (m, 1H), 4.15 - 4.06 (m, 1H), 4.07 - 3.90 (m, 2H), 2.42 (s, 3H), 2.38-2.15 (m, 2H), 1.73 - 1.45 (m, 3H), 1.05 (t, J = 7.1 Hz, 3H).

**¹³C-NMR (75 MHz, CDCl₃):** δ / ppm = 167.3, 143.2, 141.0, 135.5, 133.1, 130.5, 127.1, 126.0, 125.6, 60.2, 35.8, 29.2, 26.0, 19.5, 17.6, 14.1.

**IR (Diamond-ATR, neat):** *ν̃*/cm⁻¹ = 2936, 2867, 1712, 1692, 1647, 1603, 1488, 1463, 1446, 1424, 1390, 1369, 1344, 1288, 1238, 1168, 1146, 1096, 1081, 1059, 1018, 974, 932, 915, 882, 862, 842, 782, 756, 728, 699.

**MS (EI, 70 eV):** *m*/*z* (%) = 244 (M+, 50), 198 (100), 183 (75), 170 (42), 155 (25), 141 (33), 128 (35), 115 (27), 105 (23), 91 (17), 79 (15).

**HRMS (EI):** *m*/*z* calc. for [C₁₆H₂₀O₂]: 244.1463; found: 244.1456 (M⁺).

### 3'-(Trifluoromethyl)-1,2,3,4-tetrahydro-1,1'-biphenyl (4c)

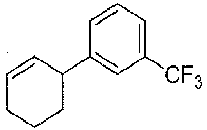

According to **TP2** cyclohex-2-en-1-ylzinc pivalate **1a** in dry THF (0.31 M, 1.60 mL, 0.50 mmol) was added to 1-bromo-3-(trifluoromethyl)benzene (90 mg, 0.40 mmol) and PEPPSI-IPent (8 mg, 0.01 mmol) in THF (0.5 mL) and the mixture was stirred for 4 h at 50 °C. Purification of the crude product by flash chromatography (silica gel, isohexane/EtOAc = 100:1) afforded the title compound as a colourless oil (65 mg, 0.29 mmol, 72%).

**¹H-NMR (300 MHz, CDCl₃):** δ / ppm = 7.50 - 7.43 (m, 2H), 7.43 - 7.37 (m, 2H), 6.00 - 5.89 (m, 1 H), 5.75 - 5.64 (m, 1 H), 3.54 - 3.40 (m, 1 H), 2.19 - 1.92 (m, 3H), 1.85 - 1.44 (m, 3H). **¹³C-NMR (75 MHz, CDCl₃):** δ / ppm = 147.7, 131.3 (q, J = 1.4 Hz), 130.7 (d, J = 31.9 Hz), 129.4, 129.3, 128.8, 124.6 (q, J = 3.8 Hz), 124.5 (d, J = 272.3 Hz), 123.0 (q, J = 3.9 Hz), 41.8, 32.7, 25.1, 21.1.

**IR (Diamond-ATR, neat):** *ν̃*/cm⁻¹ = 3406, 2943, 2876, 1658, 1613, 1491, 1435, 1327, 1230, 1163, 1118, 1098, 1072, 1001, 960, 891, 800, 760, 696, 678, 654.

**MS (EI, 70 eV):** *m*/*z* (%) = 226 (M+, 100), 211 (61), 198 (18), 185 (10), 172 (25), 157 (11), 142(9), 129(65), 115(10).

**HRMS (EI):** *m*/*z* calc. for [C₁₃H₁₃F₃]: 226.0969; found: 266.0955 (M⁺).

### 1-Methyl-4-(3-methylbut-2-en-1-yl)benzene (4d)

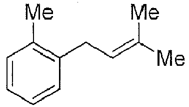

According to **TP2** prenylzinc pivalate **1 b** in dry THF (0.38 M, 2.60 mL, 1.00 mmol) was added to 2-bromotoluene (136 mg, 0.80 mmol) and PEPPSI-IPent (16 mg, 0.02 mmol) in THF (0.5 mL) and the mixture was stirred for 4 h at 50 °C. Purification of the crude product by flash chromatography (silica gel, isohexane/EtOAc = 100:1) afforded the title compound as a colourless oil (90 mg, 0.56 mmol, 70%).

**¹H-NMR (300 MHz, CDCl₃):** δ / ppm = 7.22 - 7.08 (m, 4H), 5.34 - 5.22 (m, 1 H), 3.34 (d, J = 7.3 Hz, 2H), 2.33 (s, 3H), 1.81 - 1.71 (m, 6H).

**¹³C-NMR (75 MHz, CDCl₃):** δ/ppm = 140.1, 136.3, 132.5, 130.2, 128.7, 126.1, 126.0, 122.7, 32.3, 25.9, 19.6, 18.0.

**IR (Diamond-ATR, neat):** *ν̃*/cm⁻¹ = 3414, 3066, 2974, 2930, 1716, 1603, 1576, 1491, 1459, 1380, 1286, 1251, 1220, 1160, 1142, 1075, 1052, 905, 863, 739, 692.

**MS (EI, 70 eV):** *m*/*z* (%) = 160 (M+, 57), 145 (100), 130 (22), 117 (18), 104 (23), 91 (13), 77 (8).

**HRMS (EI):** *m*/*z* calc. for [C₁₂H₁₆]: 160.1252; found: 160.1245 (M⁺).

### 1-Methoxy-3-(3-methylbut-2-en-1-yl)benzene (4e)

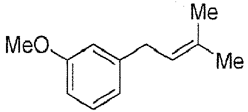

According to **TP2** prenylzinc pivalate **1 b** in dry THF (0.38 M, 2.60 mL, 1.00 mmol) was added to 3-bromoanisol (150 mg, 0.80 mmol) and PEPPSI-IPent (16 mg, 0.02 mmol) in THF (0.5 mL) and the mixture was stirred for 4 h at 50 °C. Purification of the crude product by flash chromatography (silica gel, isohexane/EtOAc = 80:1) afforded the title compound as a colourless oil (100 mg, 0.57 mmol, 71%).

**¹H-NMR (300 MHz, CDCl₃):** δ / ppm = 7.26 - 7.14 (m, 1 H), 6.84 - 6.67 (m, 3H), 5.41 - 5.25 (m, 1 H), 3.80 (s, 3H), 3.38 - 3.27 (m, 2H), 1.80 - 1.66 (m, 6H).

**¹³C-NMR (75 MHz, CDCl₃):** δ/ppm = 159.8, 143.6, 132.8, 129.4, 123.1, 120.9, 114.3, 111.1, 55.3, 34.5, 25.9, 18.0.

**IR (Diamond-ATR, neat):** *ν̃*/cm⁻¹ = 3055, 3000, 2937, 2834, 1597, 1573, 1475, 1464, 1410, 1290, 1278, 1231, 1202, 1168, 1091, 1055, 1046, 1029, 992, 908, 872, 851, 799, 771, 693. **MS (EI, 70 eV):** *m*/*z* (%) = 176 (M+, 88), 161 (100), 146 (16), 129 (13), 121 (24), 115 (14), 108(11), 91 (24), 77 (9).

**HRMS (EI):** *m*/*z* calc. for [C₁₂H₁₆O]: 176.1201; found: 176.1186 (M⁺).

### (1 R,5S)-2-(3-Fluorobenzyl)-6,6-dimethylbicyclo[3.1.1]hept-2-ene (4f)

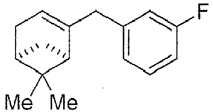

According to **TP2** (-)-myrtenylzinc pivalate **1c** in dry THF (0.32 M, 1.88 mL, 0.60 mmol) was added to 1-bromo-3-fluorobenzene (77 mg, 0.50 mmol) and PEPPSI-IPent (8 mg, 0.01 mmol) in THF (0.5 mL) and the mixture was stirred for 4 h at 50 °C. Purification of the crude product by flash chromatography (silica gel, isohexane) afforded the title compound as a colourless oil (89 mg, 0.39 mmol, 77%).

**¹H-NMR (400 MHz, CDCl₃):** δ / ppm = 7.2 - 7.1 (m, 1 H), 6.9 - 6.8 (m, 1 H), 6.8 - 6.7 (m, 2H), 5.2 - 5.2 (m, 1 H), 3.3 - 3.1 (m, 2H), 2.3 - 2.1 (m, 4H), 2.1 - 2.0 (m, 1 H), 1.9 (td, J = 5,6, 1.5 Hz, 1 H), 1.1 (s, 3H), 0.7 (s, 3H).

**¹³C**-**NMR (101 MHz, CDCl₃):** δ / ppm = 163.0 (d, J = 244.9 Hz), 146.8, 142.4 (d, J = 7.1 Hz), 129.5 (d, J = 8.2 Hz), 124.9 (d, J = 2.7 Hz), 118.4, 116.1 (d, J = 20.9 Hz), 112.9 (d, J = 21.1 Hz), 45.6, 43.4 (d, J = 1.7 Hz), 40.8, 38.0, 32.0, 31.5, 26.3, 21.1.

**IR (Diamond-ATR, neat):** *ν̃*/cm⁻¹ = 2984, 2916, 2833, 1615, 1588, 1487, 1469, 1446, 1382, 1366, 1266, 1250, 1221, 1204, 1135, 1074, 982, 949, 930, 886, 872, 806, 774, 744, 711, 701, 680.

**MS (EI, 70 eV):** *m*/*z* (%) = 230 (M+, 12), 215 (5), 186 (61), 173 (7), 159 (10), 147 (10), 133 (9), 121 (15), 109 (100), 91 (27), 79 (12), 65 (3).

**HRMS (EI):** *m*/*z* calc. for [C₁₆H₁₉F]: 230.1471; found: 230.1461 (M⁺).

### 3-Cinnamylpyridine (4g)

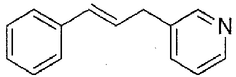

According to **TP2** cinnamylzinc pivalate **1d** in dry THF (0.57 M, 0.88 mL, 0.50 mmol) was added to 3-bromopyridine (69 mg, 0.40 mmol) and PEPPSI-IPent (8 mg, 0.01 mmol) in THF (0.5 mL) and the mixture was stirred for 4 h at 50 °C. Purification of the crude product by flash chromatography (silica gel, isohexane/EtOAc = 20:1) afforded the title compound as a colourless oil (56 mg, 0.29 mmol, 72%).

**¹H-NMR (400 MHz, CDCl₃):** δ / ppm = 8.6 - 8.5 (m, 1 H), 8.5 - 8.4 (m, 1 H), 7.2 - 7.1 (m, 4H), 7.1 - 6.9 (m, 2H), 6.8 - 6.6 (m, 1 H), 6.3 - 6.1 (m, 1H), 6.0 - 5.9 (m, 1 H), 3.1 - 3.0 (m, 2H). **¹³C-NMR (101 MHz, CDCl₃):** δ / ppm = δ 150.8, 148.3, 137.6, 135.6, 135.5, 132.2, 128.8, 128.1, 127.6, 126.6, 123.3, 36.5.

**IR (Diamond-ATR, neat):** *ν̃*/cm⁻¹ = 3028, 2958, 2924, 1679, 1596, 1578, 1557, 1495, 1479, 1449, 1424, 1372, 1272, 1190, 1125 1103, 1076, 1045, 1027, 1002, 967, 920, 745, 693. **MS (EI, 70 eV):** *m*/*z* (%) = 195 (100, M⁺), 180 (17), 167 (14), 139 (6), 115 (17), 91 (12), 77 (6).

**HRMS (EI):** *m*/*z* calc. for [C₁₄H₁₃N]: 195.2597; found: 195.1034 (M⁺).

### Diastereoselective Preparation of Homoallylic Alcohols of Type 6

### (S*)-1-(4-Chlorophenyl)-1-((R*)-cyclohex-2-en-1-yl)ethanol (6a)

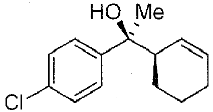

According to **TP3** cyclohex-2-en-1-ylzinc pivalate **1a** (7.10 mL, 0.28 M, 2.00 mmol) was added to a solution of 1-(4-chlorophenyl)ethanone (247 mg, 1.60 mmol) at -78 °C and the mixture was stirred 1 h at -78 °C before it was slowly warmed to room temperature overnight. Purification of the crude product by flash chromatography (silica gel, isohexane/EtOAc = 20:1) afforded the title compound as a colourless oil (328 mg, 1.47 mmol, 92%, d.r. > 99:1).

**¹H-NMR (300 MHz, CDCl₃):** 5 / ppm = 7.38 - 7.31 (m, 2H), 7.31 - 7.23 (m, 2H), 5.98 - 5.87 (m, 1 H), 5.80 - 5.70 (m, 1 H), 2.56 - 2.43 (m, 1 H), 2.00 - 1.87 (m, 2H), 1.78 - 1.63 (m, 2H), 1.56 (s, 3H), 1.48 - 1.32 (m, 2H), 1.30 - 1.16 (m, 1 H).

**¹³C**-**NMR (75 MHz, CDCl₃):** δ / ppm = 141.1, 138.2, 129.6, 128.0, 126.4, 116.6, 74.9, 72.7, 55.9, 30.6, 30.6, 22.2, 21.1, 0.7.

**IR (Diamond-ATR, neat):** *ν̃*/cm⁻¹ = 3571, 3455, 3026, 2931, 2860, 2837, 1597, 1490, 1448, 1398, 1372, 1343, 1307, 1257, 1176, 1093, 1044, 1038, 1012, 904, 892, 879, 860, 833, 772, 756, 724, 688.

**MS (EI, 70 eV):** *m*/*z* (%) = 218 (1), 155 (100), 138 (13), 111 (10), 77 (11), 53 (5).

HRMS **(EI):** *m*/*z* calc. for [C₁₄H₁₅Cl]: 218.0862; found: 218.0853 ([M-H₂O]⁺).

### (S*)-1-(4-Fluorophenyl)-1-((R*)-cyclohex-2-en-1-yl)ethanol (6b)

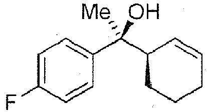

According to **TP3** cyclohex-2-en-1-ylzinc pivalate **1a** (4.29 mL, 0.28 M, 1.20 mmol) was added to a solution of 1-(4-fluorophenyl)ethanone (247 mg, 1.60 mmol) at -78 °C and the mixture was stirred 1 h at -78 °C. Purification of the crude product by flash chromatography (silica gel, isohexane/EtOAc = 20:1) afforded the title compound as a colourless oil (196 mg, 0.890 mmol, 89%, d.r. > 99:1).

**¹H-NMR (300 MHz, CDCl₃):** δ / ppm = 7.46 - 7.32 (m, 2H), 7.09 - 6.94 (m, 2H), 5.99 - 5.85 (m, 1 H), 5.82 - 5.70 (m, 1 H), 2.58 - 2.44 (m, 1 H), 2.00 - 1.89 (m, 2H), 1.79 - 1.65 (m, 2H), 1.58 (s, 3H), 1.52 - 1.33 (m, 2H), 1.33 - 1.16 (m, 1 H).

**¹³C**-**NMR (75 MHz, CDCl₃):** δ / ppm = 161.7 (d, J = 244.6 Hz), 143.0 (d, J = 3.0 Hz), 132.0, 127.0 (d, J = 7.8 Hz), 126.3, 114.7 (d, J = 21.1 Hz), 75.9, 46.8, 28.2, 25.3, 24.5, 22.0.

**IR (Diamond-ATR, neat):** *ν̃*/cm⁻¹ = 3406, 3027, 2933, 2863, 1660, 1601, 1508, 1450, 1408, 1373, 1345, 1306, 1222, 1160, 1112, 1081, 1014, 965, 942, 924, 906, 892, 880, 836, 821, 794, 753, 735, 727, 716.

**MS (EI, 70 eV):** *m*/*z* (%) = 202 (1), 159 (2), 146 (2), 139 (100), 123 (14), 109 (3), 95 (7), 79 (5).

**HRMS (EI):** *m*/*z* calc. for [C₁₄H₁₅F]: 202.1158; found: 202.1132 ([M-H₂O]⁺).

### (R*)-1-((R*)-cyclohex-2-en-1-yl)-1-cyclopropylethanol (6c)

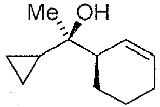

According to **TP3** cyclohex-2-en-1-ylzinc pivalate **1a** (5.30 mL, 0.23 M, 1.20 mmol) was added to a solution of cyclopropyl methyl ketone (84 mg, 1.00 mmol) at -78 °C and the mixture was stirred 1.5 h at -78 °C. Purification of the crude product by flash chromatography (silica gel, isohexane/EtOAc = 5:1) afforded the title compound as a colourless oil (95 mg, 0.57 mmol, 92%, d.r. = 94:6).

**¹H-NMR (400 MHz, CDCl₃):** δ / ppm = 5.90-5.77 (m, 2H), 2.36-2.26 (m, 1 H), 2.05-1.94 (m, 2H),

1.94-1.76 (m, 2H), 1.55-1.37 (m, 2H), 1.10 (s, 1H), 1.02 (s, 3H), 1.01-0.90 (m, 1H), 0.47-0.25 (m, 4H).

**¹³C-NMR (101 MHz, CDCl₃):** δ / ppm = 129.5, 127.9, 73.3, 47.5, 25.4, 24.5, 23.3, 22.4, 19.3, 1.7, 0.5.

**IR (Diamond-ATR, neat)**: *ν̃*/cm⁻¹ = 3448, 3083, 3027, 2971, 2929, 1700, 1684, 1653, 1559, 1576, 1522, 1507, 1456, 1448, 1435,1371, 1308, 1272, 1254, 1201, 1178, 1141, 1106, 1078, 1050, 1035, 1020, 998, 929, 910, 876, 827, 809, 789, 721, 672, 650.

**MS (EI, 70 eV):** *m*/*z* (%) = 166 (M+, 1), 148 (45), 133 (45), 119 (40), 105 (100), 91 (100), 79 (80).

**HRMS (EI):** *m*/*z* calc. for [C₁₁H₁₇]: 149.1330; found: 149.2278 ([M-H₂O]⁺).

### 1,1,1-Trifluoro-3,3-dimethyl-2-phenylpent-4-en-2-ol (6d)

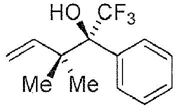

According to **TP3** prenylzinc pivalate **1 b** (3.50 mL, 0.32 M, 1.10 mmol) was added to a solution of 2,2,2-trifluoro-1-phenylethanone (84 mg, 1.00 mmol) at -78 °C and the mixture was stirred 1.5 h at -78 °C before it was slowly warmed to room temperature overnight. Purification of the crude product by flash chromatography (silica gel, isohexane/EtOAc = 30:1) afforded the title compound as a colourless oil (212 mg, 0.87 mmol, 87%).

**¹H-NMR (300 MHz, CDCl₃):** δ / ppm = 7.61 (d, J = 5.2 Hz, 2H), 7.37 (m, 3H), 6.40-5.78 (m, 1 H),

5.20 (m, 2H), 2.80 (s, 1 H), 1.20 (s, 3H), 0.97 (s, 3H).

**¹³C**-**NMR (75 MHz, CDCl₃):** δ / ppm = 164.2, 143.3 (q, J = 1.6 Hz), 128.3, 127.7, 127.2 (q, J = 2.0

Hz), 126.6 (q, J = 288.8 Hz), 115.7, 80.4 (q, J = 25.9 Hz), 44.3, 23.8 (q, J = 1.8 Hz), 23.0 (q, J= 1.8 Hz).

**IR (Diamond-ATR, neat):** *ν̃*/cm⁻¹ = 3417 (vw), 2981 (vw), 2254 (vw), 1708 (m), 1639 (vw), 1497 (vw), 1477 (vw), 1449 (w), 1416 (w), 1364 (m), 1288 (w), 1265 (m), 1225 (m), 1146 (s), 1100

(w), 1068 (m), 1035 (w), 1009 (w), 967 (w), 907 (s), 847 (vw), 812 (vw), 760 (m), 725 (vs), 702 (s).

**MS (EI, 70 eV):** *m*/*z* (%) = 175 (95), 127 (15), 105 (100), 91 (7), 77 (42), 69 (93), 51 (14).

### (R)-((1R,3R,5R)-6,6-dimethyl-2-methylenebicyclo[3.1.1]heptan-3-yl)(furan-2-yl)methanol (6e)

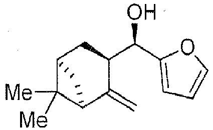

According to **TP3** (-)-myrtenylzinc pivalate **1c** (3.76 mL, 0.32 M, 1.20 mmol) was added to a solution of cyclopropyl methyl ketone (96 mg, 1.00 mmol) at -78 °C and the mixture was stirred 1 h at -78 °C. Purification of the crude product by flash chromatography (silica gel, isohexane/EtOAc = 10:1) afforded the title compound as a colourless oil (206 mg, 0.89 mmol, 89%, d.r. > 99:1).

**¹H**-**NMR (600 MHz, CDCl₃):** δ / ppm = 7.4 (dd, J = 1.9, 0.9 Hz, 1H), 6.3 (dd, J = 3.3, 1.8 Hz, 1H), 6.3 - 6.3 (m, 1 H), 4.9 (dt, J = 7.9, 1.7 Hz, 2H), 4.5 (dd, J = 9.4, 2.4 Hz, 1 H), 3.0 - 2.9 (m, 1 H), 2.9 (d, J = 2.6 Hz, 1 H), 2.5 (t, J = 5.4 Hz, 1 H), 2.3 - 2.2 (m, 1H), 2.0 - 1.9 (m, 2H), 1.6 - 1.5 (m, 1 H), 1.3 (s, 3H), 1.2 (d, J = 10.3 Hz, 1 H), 0.8 (s, 3H).

**¹³C-NMR (151 MHz, CDCl₃**)**:** δ / ppm = 155.0, 151.7, 142.2, 111.8, 110.3, 108.1, 72.3, 52.5, 41.4, 40.6, 40.3, 27.5, 26.7, 25.9, 21.7.

**IR (Diamond-ATR, neat):** *ν̃*/cm⁻¹ = 3417, 2978, 2918, 2869, 1695, 1635, 1504, 1456, 1383, 1368, 1316, 1262, 1224, 1211, 1186, 1149, 1107, 1076, 1052, 1029, 1009, 956, 941, 930, 920,884,841,807,731,702,689,656.

**MS (EI, 70 eV):** *m*/*z* (%) = 232 (M+, 1), 214 (8), 199 (6), 189 (3), 171 (14), 136 (19), 121 (15), 105 (8), 97 (100), 93 (66), 77 (16).

**HRMS (EI):** *m*/*z* calc. for [C₁₅H₂₀O₂]: 232.1463; found: 232.1467 (M⁺).

### (1R*,2R*)-1-(3-Bromophenyl)-2-phenylbut-3-en-1-ol (6f)

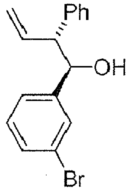

According to **TP3** cinnamylzinc pivalate **1d** (1.01 g, 0.95 mmol/g, 0.96 mmol) was solved in 3 mL THF. 3-Bromobenzaldehyde (142 mg, 0.77 mmol) was added at -78 °C and the mixture was stirred 1 h at -78 °C. Purification of the crude product by flash chromatography (silica gel, isohexane/EtOAc = 10:1) afforded the title compound as a colourless oil (201 mg, 0.663 mmol, 86%).

**¹H-NMR (300 MHz, CDCl₃):** δ / ppm = 7.48 - 7.10 (m, 5H), 7.10 - 6.92 (m, 4H), 6.31 - 6.12 (m, 1H), 5.41 - 5.14 (m, 2H), 4.79 (d, J = 7.6 Hz, 1H), 3.53 - 3.42 (m, 1H), 2.33 (s, 1H). **¹³C-NMR (75 MHz, CDCl₃):** δ / ppm = 144.1, 140.1, 137.2, 130.4, 129.6, 129.3, 128.5, 128.2, 126.8, 125.4, 122.1, 118.9, 76.5, 59.2.

**IR (Diamond-ATR, neat):** cm⁻¹ = 3537, 3415, 3062, 3029, 2899, 1684, 1638, 1596, 1570, 1493, 1475, 1453, 1428, 1287, 1184, 1094, 1070, 1032, 996, 920, 849, 783, 757, 728, 696, 674.

**MS (EI, 70 eV):** *m*/*z* (%) = 183 (67), 155 (30), 131 (3), 117 (100), 103 (10), 91 (27), 77 (28), 63 (13), 51 (28).

**HRMS (EI):** *m*/*z* calc. for [C₁₆H₁₃Br]: 284.0201; found: 284.0178 ([M-H₂O]⁺).

### Trimethyl(((1R,2S)-2-((R)-1-phenylallyl)cyclohexyl)oxy)silane (6g)

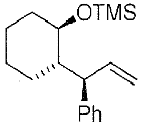

According to **TP3** cinnamylzinc pivalate **1d** (3.00 mL, 0.16 M, 0.48 mmol) was added to a solution of 2-((trimethylsilyl)oxy)cyclohexanone (83 mg, 0.40 mmol) at -78 °C and the mixture was stirred 1 h at -78 °C. Purification of the crude product by flash chromatography (silica gel, isohexane/EtOAc = 100:1) afforded the title compound as a colourless oil (92 mg, 0.31 mmol, 80%, d.r. > 99:1).

**¹H-NMR (300 MHz, CDCl₃):** δ / ppm = 7.34 - 7.14 (m, 5H), 6.47 - 6.25 (m, 1 H), 5.20 - 4.95 (m, 2H), 3.77 - 3.56 (m, 1 H), 3.56 - 3.42 (m, 1 H), 2.35 (s, 1 H), 1.79 - 1.43 (m, 6H), 1.36-1.25 (m, 1H), 1.17 - 1.03 (m, 1H), 0.14 (s, 9H).

**¹³C**-**NMR (75 MHz, CDCl₃):** δ / ppm = 141.1, 138.2, 129.6, 128.0, 126.4, 116.6, 74.9, 72.7, 55.9, 30.6, 30.6, 22.2, 21.1, 0.7.

**IR (Diamond-ATR, neat):** *ν̃*/cm⁻¹ = 3565, 3426, 3078, 3029, 2948, 2930, 2861, 1630, 1493, 1452, 1446, 1401, 1359, 1346, 1321, 1253, 1150, 1088, 1046, 1032, 1008, 975, 967, 953, 910, 893, 840, 740, 705.

**MS (EI,** 70 eV): *m*/*z* (%) = 187 (29), 171 (100), 155 (2), 141 (7), 129 (15), 117 (34), 103 (8), 91 (15), 73 (31).

**HRMS (EI):** *m*/*z* calc. for [C₁₅H₁₈O]: 214.1358; found: 214.1358 ([M-TMSH]⁺).

### (R*)-ethyl 5-((S*)-(3-bromophenyl)(hydroxy)methyl)cyclopent-1-enecarboxylate (6h)

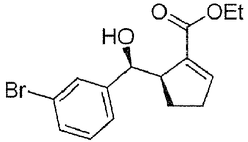

According to **TP3** (2-(ethoxycarbonyl)cyclopent-2-en-1-yl)zinc pivalate **1e** (5.22 mL, 0.23 M, 1.20 mmol) was added to a solution of 3-bromobenzaldehyde (185 mg, 1.00 mmol) at -78 °C and the mixture was stirred 1 h at -78 °C. Purification of the crude product by flash chromatography (silica gel, isohexane/EtOAc = 5:1) afforded the title compound as a colourless oil (294 mg, 0.91 mmol, 91%, d.r. = 97:3).

**¹H**-**NMR** (**400 MHz, CDCl₃):** δ / ppm = 7.5 - 7.5 (m, 1 H), 7.4 - 7.3 (m, 1 H), 7.3 - 7.2 (m, 1 H), 7.2 - 7.1 (m, 1 H), 6.9 - 6.9 (m, 1 H), 5.1 (d, J = 3.3 Hz, 1 H), 4.3 - 4.2 (m, 2H), 3.5 - 3.3 (m, 1 H), 3.1 (s, 1 H), 2.4 - 2.2 (m, 2H), 2.0 - 1.8 (m, 2H), 1.3 (t, J = 7.1 Hz, 3H).

**¹³C**-**NMR (101 MHz, CDCl₃):** δ / ppm = 166.2, 148.1, 145.5, 136.0, 130.2, 129.7, 129.3, 124.8, 122.4, 73.9, 60.9, 52.0, 32.2, 24.8, 14.4.

**IR (Diamond-ATR, neat):** *ν̃*/cm⁻¹ = 3458, 2979, 2939, 2906, 1690, 1628, 1595, 1569, 1473, 1452, 1447, 1426, 1394, 1372, 1343, 1295, 1253, 1195, 1097, 1069, 1043, 1011, 980, 946, 897, 842, 785, 743, 697, 674.

**MS (EI, 70 eV):** *m*/*z* (%) = 281 (1), 261 (1), 185 (8), 171 (3), 157 (7), 140 (100), 112 (35), 94 (17), 77 (26), 67 (28).

**HRMS (EI):** *m*/*z* calc. for [C₁₅H₁₇⁸¹BrO₃]: 326.0341; found: 326.0508 (M⁺).

### (3R*,3aS*)-3-Methyl-3-ferrocene-3a,4,5,6-tetrahydroisobenzofuran-1(3H)-one (6i)

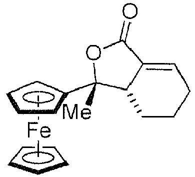

According to **TP3** ((2-(ethoxycarbonyl)cyclohex-2-en-1-yl)zinc pivalate **1f** (3.00 mL, 0.40 M, 1.20 mmol) was added to a solution of acetylferrocene (228 mg, 1.00 mmol) at -78 °C and the mixture was stirred 1 h at -78 °C before it was slowly warmed to room temperature overnight. Purification of the crude product by flash chromatography (silica gel, isohexane/EtOAc = 10:1) afforded the title compound as a yellow solid (310 mg, 0.92 mmol, 92%, d.r. > 99:1).

**¹H-NMR (300 MHz, CDCl₃):** δ / ppm = 6.9 - 6.7 (m, 1 H), 4.2 (s, 5H), 4.2 - 4.1 (m, 2H), 4.0-3.9 (m, 1 H), 3.8 - 3.7 (m, 1H), 2.9 - 2.7 (m, 1 H), 2.3 - 2.1 (m, 1 H), 2.1 - 1.9 (m, 1 H), 1.9 (s, 3H), 1.8 - 1.5 (m, 3H), 1.5 - 1.3 (m, 1 H).

**¹³C-NMR (75 MHz, CDCl₃):** δ / ppm = 170.4, 136.5, 130.8, 91.0, 86.6, 69.2, 68.3, 67.9, 67.2, 64.7, 48.2, 26.8, 25.1, 23.8, 20.9.

**IR (Diamond-ATR, neat):** *ν̃*/cm⁻¹ = 1749, 1680, 1376, 1254, 1242, 1201, 1154, 1112, 1105, 1025, 998, 946, 932, 919, 899, 871, 862, 850, 830, 812, 791, 738, 715, 679.

**MS (EI, 70 eV):** *m*/*z* (%) = 281 (1), 261 (1), 185 (8), 171 (3), 157 (7), 140 (100), 112 (35), 94 (17), 77 (26), 67 (28).

**HRMS (EI):** *m*/*z* calc. for [C₁₉H₂₀FeO₂]: 336.0813; found: 336.0787 (M⁺).

### (S*)-5-((R*)-furan-2-yl(hydroxy)methyl)cyclopent-1-enecarbonitrile (6j)

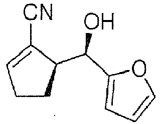

According to **TP3** (2-cyanocyclopent-2-en-1-yl)zinc pivalate **1g** (2.70 mL, 0.37 M, 1.00 mmol) was added to a solution of furan-2-carbaldehyde (77 mg, 0.80 mmol) at -78 °C and the mixture was stirred 1 h at -78 °C. Purification of the crude product by flash chromatography (silica gel, isohexane/EtOAc = 5:1) afforded the title compound as a colourless oil (138 mg, 0.73 mmol, 91%, d.r. > 99:1).

**¹H**-**NMR (400 MHz, CDCl₃):** δ / ppm = 7.5-7.4 (m, 1 H), 6.8-6.7 (m, 1 H), 6.4 (dd, J = 3.2, 1.8 Hz, 1H), 6.3 (d, J = 3.3 Hz, 1 H), 4.9 (d, J = 4.4 Hz, 1 H), 3.5 - 3.3 (m, 1 H), 2.6 - 2.4 (m, 2H), 2.2 - 2.0 (m, 3H).

**¹³C-NMR** (101 **MHz, CDCl₃**)**:** δ / ppm = 154.6, 151.9, 142.4, 116.1, 115.5, 110.5, 107.4, 68.2, 52.1, 33.1, 24.2.

**IR (Diamond-ATR, neat)**: *ν̃*/cm⁻¹ = 3436, 2949, 2220, 1732, 1691, 1616, 1504, 1454, 1432, 1375, 1324, 1224, 1149, 1072, 1006, 983, 951, 928, 916, 884, 815, 739.

**MS (EI, 70 eV):** *m*/*z* (%) = 189 (1), 115 (1), 97 (100), 81 (1), 69 (5), 51 (2).

**HRMS (EI):** *m*/*z* calc. for [C₁₁H₁₁NO₂]: 189.0790; found: 189.0800 (M⁺).

### Regioselective Preparation of β,γ-Unsaturated Ketones of Type 8

### (4-Chlorophenyl)(cyclohex-2-en-1-yl)methanone (8a)

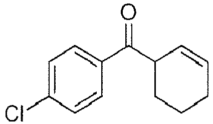

According to **TP4** cyclohex-2-en-1-ylzinc pivalate **1a** (3.45 mL, 0.29 M, 1.00 mmol) was added to a solution of 4-chlorobenzoyl chloride (350 mg, 1.50 mmol) at -78 °C and the mixture was stirred 1.5 h at -78 °C. Purification of the crude product by flash chromatography (silica gel, isohexane/EtOAc = 100:1) afforded the title compound as a colourless oil (172 mg, 0.78 mmol, 78%).

**¹H-NMR (300 MHz, CDCl₃**)**:** δ / ppm = 7.96 - 7.84 (m, 13H), 7.48 - 7.39 (m, 12H), 5.99 - 5.87 (m, 7H), 5.76 - 5.66 (m, 7H), 4.09 - 3.95 (m, 7H), 2.15 - 2.03 (m, 13H), 2.02 - 1.76 (m, 22H), 1.76 - 1.61 (m, 8H).

**¹³C-NMR (75 MHz, CDCl₃):** 5 / ppm = 200.7, 139.4, 134.7, 130.6, 130.1, 129.1, 124.5, 44.1, 25.9, 24.9, 21.0.

**IR (Diamond-ATR, neat):** *ν̃*cm⁻¹ = 3404, 3029, 2937, 2864, 2837, 1680, 1650, 1587, 1570, 1487, 1449, 1431, 1399, 1348, 1302, 1265, 1248, 1230, 1210, 1175, 1158, 1106, 1090, 1012, 961, 927, 890, 837, 798, 761, 740, 677.

**MS (EI, 70 eV):** *m*/*z* (%) = 220 (M+, 17), 185 (3), 141 (100), 111 (82), 75 (34), 50 (11). **HRMS (EI):** *m*/*z* calc. for [C₁₃H₁₄ClO⁺]: 221.0728; found: 221.0731 ([M+H]⁺).

### 1-(4-Chlorophenyl)-2,2-dimethylbut-3-en-1-one (8b)

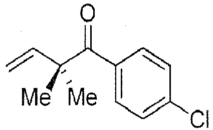

According to **TP4** prenylzinc pivalate **1b** (3.80 mL, 0.32 M, 1.20 mmol) was added to a solution of 4-chlorobenzoyl chloride (175 mg, 1.00 mmol) at -78 °C and the mixture was stirred 1 h at -78 °C before it was slowly warmed to room temperature overnight. Purification of the crude product by flash chromatography (silica gel, isohexane/EtOAc = 100:1) afforded the title compound as a colourless oil (194 mg, 0.93 mmol, 93%).

**¹H-NMR (300 MHz, CDCl₃):** δ / ppm = 7.88-7.80 (m, 2H), 7.38-7.31 (m, 2H), 6.23-6.10 (m, 1 H),

5.24 (d, J = 6.8 Hz, 1 H), 5.20 (s, 1 H), 1.38 (s, 6H).

**¹³C-NMR (75 MHz, CDCl₃):** δ / ppm = 203.3, 143.8, 138.2, 135.3, 131.0, 128.4, 114.5, 50.3, 26.1.

**IR (Diamond-ATR, neat):** / cm⁻¹ = 3539 (vw), 3084 (w), 2975 (m), 2932 (w), 2874 (vw), 1677 (vs), 1633 (m), 1587 (s), 1569 (m), 1486 (m), 1466 (m), 1443 (w), 1412 (m), 1399 (m), 1379 (m), 1363 (m), 1303 (w), 1255 (s), 1220 (m), 1170 (m), 1153 (m), 1090 (vs), 1044 (w), 1012 (s), 970 (vs), 918 (vs), 886 (w), 840 (vs), 759 (vs), 741 (w), 733 (m), 665 (m).

**MS (EI, 70 eV):** *m*/*z* (%) = 175 (95), 127 (15), 105 (100), 91 (7), 77 (42), 69 (93), 51 (14). **HRMS (EI):** *m*/*z* calc. for [C₁₂H₁₃ClO]: 208.0655; found: 208.0629 (M⁺).

### 1-(4-Methoxyphenyl)-4-methylpent-3-en-1-one (8c)

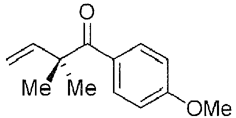

According to **TP4** prenylzinc pivalate **1 b** (0.28 M, 4.29 mL, 1.20 mmol) was added to a solution of 4-methoxybenzoyl chloride (171 mg, 0.13 mL, 1.00 mmol) at -78 °C and the mixture was stirred 1 h at -78 °C before it was slowly warmed to room temperature overnight. Purification of the crude product by flash chromatography (silica gel, isohexane/EtOAc = 40:1) afforded the title compound as a colourless oil (233 mg, 1.14 mmol, 95%).

**¹H-NMR (300 MHz, CDCl₃**)**:** δ / ppm = 7.98-7.93 (m, 2H), 6.88-6.83 (m, 2H), 6.24-6.15 (m, 1H), 5.24-5.16 (m, 2H), 3.83 (s, 3H), 1.39 (s, 6H).

**¹³C**-**NMR (75 MHz, CDCl₃):** δ / ppm = 202.5, 162.5, 144.6, 132.1, 129.3, 113.7, 113.2, 55.5, 50.0, 26.4.

**IR (Diamond-ATR, neat):** *ν̃*/cm⁻¹ = 3082, 2972, 2934, 2840, 2360, 2340, 2047, 1668, 1633, 1598, 1573, 1508, 1462, 1442, 1417, 1380, 1362, 1307, 1247, 1166, 1150, 1113, 1030, 1012, 996, 971, 917, 839, 820, 787, 767, 736, 702, 670.

**MS (EI, 70 eV):** *m*/*z* (%) = 135 (100), 107 (7), 92 (9), 77 (9).

**HRMS (ESI):** *m*/*z* calc. for [C₁₃H₁₇O₂]: 205.1229; found: 205.1213 ([M+H]⁺).

### Ethyl 5-(3-bromobenzoyl)cyclopent-1-enecarboxylate (8d)

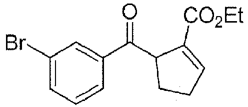

According to **TP4** (2-(ethoxycarbonyl)cyclopent-2-en-1-yl)zinc pivalate **1e** (5.00 mL, 0.30 M, 1.5 mmol) was added to a solution of 3-bromobenzoyl chloride (296 mg, 1.30 mmol) at -78 °C and the mixture was stirred 1 h at -78 °C before it was slowly warmed to room temperature overnight. Purification of the crude product by flash chromatography (silica gel, isohexane/EtOAc = 15:1) afforded the title compound as a colourless oil (297 mg, 0.92 mmol, 68%).

**¹H-NMR (599 MHz, CDCl₃):** δ / ppm = 8.13 (s, 1 H), 7.93 (d, J = 8.1, 1.4 Hz, 1 H), 7.73 - 7.67 (m, 1H), 7.39 - 7.33 (m, 1H), 7.06 - 7.00 (m, 1H), 4.78 - 4.67 (m, 1H), 4.19 - 4.06 (m, 2H), 2.76 - 2.66 (m, 1H), 2.65 - 2.56 (m, 1H), 2.49 - 2.40 (m, 1H), 2.08 - 1.99 (m, 1H), 1.18 (t, J = 7.1 Hz, 3H).

**¹³C**-**NMR (151 MHz, CDCl₃):** δ / ppm = 199.9, 164.1, 146.3, 138.3, 135.8, 131.6, 130.1, 127.1, 122.9, 60.4, 51.4, 32.8, 29.13, 14.1.

**IR (Diamond-ATR, neat):** /cm⁻¹ = 2982, 2936, 1721, 1687, 1638, 1566, 1467, 1446, 1421, 1372, 1296, 1280, 1262, 1208, 1174, 1145, 1100, 1070, 1025, 998, 956, 862, 799, 754, 732, 706, 698, 673.

**MS (EI, 70 eV):** *m*/*z* (%) = 322 (1), 278 (30), 183 (100), 155 (21), 139 (2), 93 (4), 76 (10). **HRMS (EI):** *m*/*z* calc. for [C₁₃H₁₀BrO₂⁺]: 276.9864; found: 276.9853 ([M-OEt]⁺).

### Ethyl 5-(4-(tert-butyl)benzoyl)cyclopent-1-ene-1-carboxylate (8e)

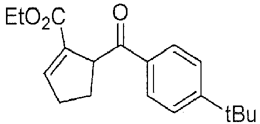

According to **TP4** (2-cyanocyclohex-2-enyl)zinc pivalate **1e** (0.21 M, 4.76 mL, 1.00 mmol) was added to a solution of 4-(tert-butyl)benzoyl chloride (295 mg, 0.29 mL, 1.50 mmol) at -78 °C and the mixture was stirred 1 h at -78 °C before it was slowly warmed to room temperature overnight. Purification of the crude product by flash chromatography (silica gel, isohexane/EtOAc = 40:1) afforded the title compound as a colourless oil (225 mg, 0.75 mmol, 75%).

**¹H-NMR (300 MHz, CDCl₃):** δ / ppm = 7.98-7.93 (m, 2H), 7.51-7.46 (m, 2H), 7.03-7.00 (m, 1 H), 4.82-4.74 (m, 1 H), 4.12 (q, J = 7.1 Hz, 2H), 2.74-2.56 (m, 2H), 2.12-1.98 (m, 2H), 1.35 (s, 9H), 1.17 (t, *J =* 7.1 Hz, 3H).

**¹³C-NMR (75 MHz, CDCl₃):** δ / ppm = 200.9, 164.5, 156.9, 146.1, 136.4, 134.0, 128.8, 125.6, 60.4, 51.5, 35.2, 32.9, 31.2, 29.5, 14.2.

**IR (Diamond-ATR, neat):** *ν̃*/cm⁻¹ = 2963 (m), 2871 (w), 1712 (vs), 1679 (vs), 1635 (w), 1604 (m), 1566 (w), 1463 (m), 1408 (m), 1394 (w), 1369 (m), 1331 (m), 1269 (vs), 1222 (vs), 1188 (s), 1144 (m), 1096 (vs), 1031 (s), 996 (s), 951 (w), 923 (m), 878 (m), 849 (s), 829 (m), 761 (m), 732 (s), 702 (m).

### 6-(Thiophene-2-carbonyl)cyclohex-1-enecarbonitrile (8f)

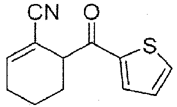

According to **TP4** (2-Cyanocyclohex-2-enyl)zinc pivalate **1e** (4.50 mL, 0.22 M, 1.00 mmol) was added to a solution of thiophene-2-carbonyl chloride (132 mg, 0.90 mmol) at -78 °C and the mixture was stirred 1 h at -78 °C before it was slowly warmed to room temperature overnight. Purification of the crude product by flash chromatography (silica gel, isohexane/EtOAc = 5:1) afforded the title compound as a colourless oil (150 mg, 0.69 mmol, 77%). **M.p. (°C):** 78.5-79.3.

**¹H-NMR (599 MHz, CDCl₃**)**:** δ / ppm = 7.78 (dd, J = 3.8, 1.0 Hz, 1H), 7.71 (dd, J = 4.9, 1.1 Hz, 1H), 7.17 (dd, J = 4.9, 3.9 Hz, 1 H), 6.93 (td, J = 4.1, 1.5 Hz, 1 H), 4.20-4.06 (m, 1 H), 2.38-2.18 (m,2H), 2.14-1.94 (m, 2H), 1.84-1.64 (m, 2H).

**¹³C**-**NMR (151 MHz, CDCl₃): δ** / ppm = 191.3, 148.8, 142.6, 134.9, 132.8, 128.5, 118.7, 110.9,

45.7, 26.8, 25.7, 18.5.

**IR (Diamond-ATR, neat):** / cm⁻¹ = 3113, 3092, 2934, 2862, 2215, 1645, 1518, 1449, 1412, 1353, 1336, 1317, 1288, 1248, 1213, 1151, 1127, 1078, 1046, 995, 952, 941, 926, 888, 858, 828, 794, 762, 736, 665.

**MS (EI, 70 eV):** *m*/*z* (%) = 217.0 (5), 110.9 (100), 83.0 (10).

**HRMS (EI):** *m*/*z* calc. for [C₁₂H₁₁ONS]: 217.0561; found: 217.0568 (M⁺).

## Claims

1. An organozinc composition comprising:
(a) an organozinc complex, wherein a zinc atom carries
(a1) a ligand X¹ selected from F, Cl, Br, I, phosphate and sulfonate, and
(a2) an organyl ligand R¹ containing at least one non-aromatic carbon-carbon double bond, wherein a coordinative bond between the zinc atom and the ligand R¹ is formed in allylic position relative to the non-aromatic carbon-carbon double bond of the ligand R¹;
(b) Mg(R²)₂, wherein the groups R² are independently selected from carboxylic acid anions; and
(c) LiX², wherein X² is selected from Cl, Br and I.

2. The organozinc composition of claim 1 which is a solid composition.

3. The organozinc composition of claim 1 or 2, wherein both groups R² are carboxylic acid anions having a tertiary carbon atom in α-position to the carboxylate group.

4. The organozinc composition of any of claims 1 to 3, wherein the ligand R¹ is a ligand of formula (I): 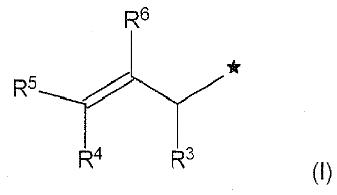 wherein the star * marks the coordinative bond to the zinc atom,
R³ to R⁶ are independently selected from hydrogen, optionally substituted alkyl, optionally substituted cycloalkyl, optionally substituted alkenyl, optionally substituted cycloalkenyl, optionally substituted alkinyl, optionally substituted alkoxy, optionally substituted aryl, optionally substituted heteroaryl, -COOR⁷, -NR⁷R⁸, -SiR⁷R⁸R⁹, F and CN, wherein R⁷ to R⁹ are independently selected from hydrogen, optionally substituted alkyl, optionally substituted cycloalkyl, optionally substituted alkenyl, optionally substituted cycloalkenyl, optionally substituted aryl and optionally substituted heteroaryl;
wherein any optionally substituted alkyl, optionally substituted cycloalkyl, optionally substituted alkenyl, optionally substituted cycloalkenyl, optionally substituted alkinyl, and optionally substituted alkoxy may be substituted by one or more substituents independently selected from alkoxy, aryl, -COOR^{7a}, -NR^{7a}R^{8a}, F, and CN, wherein R^{7a} and R^{8a} are independently selected from hydrogen, alkyl, cycloalkyl, alkenyl, cycloalkenyl and aryl; wherein any optionally substituted aryl and optionally substituted heteroaryl may be substituted by one or more substituents independently selected from alkyl, alkenyl, alkinyl, alkoxy, -COOR^{7a}, -NR^{7a}R^{8a}, F, and CN, wherein R^{7a} and R^{8a} are independently selected from hydrogen, alkyl, cycloalkyl, alkenyl, cycloalkenyl and aryl;
and wherein any two suitable groups selected from R³ to R⁶ may be linked to each other such that a group is formed which comprises one or more cyclic moieties.

5. A process for the preparation of the organozinc composition as defined in any one of claims 1 to 4, said process comprising:
reacting (i) elemental zinc with (ii) an organic starting compound containing at least one non-aromatic carbon-carbon double bond and a substituent X¹ selected from F, Cl, Br, I, phosphate and sulfonate, which substituent X¹ is bound to a carbon atom in allylic position relative to the non-aromatic carbon-carbon double bond,
to yield the organozinc complex (a) wherein a zinc atom carries a ligand X¹ corresponding to said substituent X¹ and an organyl ligand R¹ containing at least one non-aromatic carbon-carbon double bond, and a coordinative bond between the zinc atom and R¹ is formed in allylic position relative to the non-aromatic carbon-carbon double bond;
wherein the reaction of the elemental zinc and the organic starting compound is carried out in the presence of LiX², wherein X² is selected from Cl, Br and I; and
wherein Mg(R²)₂ is provided in the reaction system either prior to, during or after the reaction of the elemental zinc with the organic starting compound, with the groups R² being independently selected from carboxylic acid anions.

6. The process of claim 5, wherein the Mg(R²)₂ is provided in the reaction system by adding it to the reaction system prior to the reaction of elemental zinc with the organic starting compound, and the reaction is carried out in the presence of Mg(R²)₂, and wherein R² is defined as in claim 5.

7. The process of claim 5, wherein the Mg(R²)₂ is provided by adding Mg(X²)₂ and LiR² to the reaction system prior to the reaction of elemental zinc with the organic starting compound, so that Mg(R²)₂ and LiX² are formed in the reaction system *in-situ,* wherein R² and X² are defined as in claim 5.

8. The process of any of claims 5 to 7, wherein the organic starting compound is a compound of formula (II): 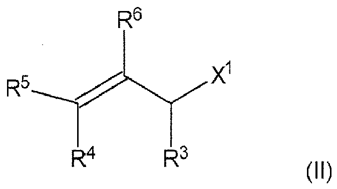 wherein
R³ to R⁶ are independently selected from hydrogen, optionally substituted alkyl, optionally substituted cycloalkyl, optionally substituted alkenyl, optionally substituted cycloalkenyl, optionally substituted alkinyl, optionally substituted alkoxy, optionally substituted aryl, optionally substituted heteroaryl, -COOR⁷, -NR⁷R⁸, -SiR⁷R⁸R⁹, F and CN, wherein R⁷ to R⁹ are independently selected from hydrogen, optionally substituted alkyl, optionally substituted cycloalkyl, optionally substituted alkenyl, optionally substituted cycloalkenyl, optionally substituted aryl, and optionally substituted heteroaryl;
wherein any optionally substituted alkyl, optionally substituted cycloalkyl, optionally substituted alkenyl, optionally substituted cycloalkenyl, optionally substituted alkinyl, and optionally substituted alkoxy may be substituted by one or more substituents independently selected from alkoxy, aryl, -COOR^{7a}, -NR^{7a}R^{8a}, F, and CN, wherein R^{7a} and R^{8a} are independently selected from hydrogen, alkyl, cycloalkyl, alkenyl, cycloalkenyl and aryl, wherein any optionally substituted aryl and optionally substituted heteroaryl may be substituted by one or more substituents independently selected from alkyl, alkenyl, alkinyl, alkoxy, -COOR^{7a}, -NR^{7a}R^{8a}, F, and CN, wherein R^{7a} and R^{8a} are independently selected from hydrogen, alkyl, cycloalkyl, alkenyl, cycloalkenyl and aryl;
and wherein any two suitable groups selected from R³ to R⁶ may be linked to each other such that a group is formed which comprises one or more cyclic moieties;
and wherein X¹ is selected from F, Cl, Br, I, phosphate and sulfonate.

9. Use of the organozinc composition of any of claims 1 to 4 in chemical synthesis as a reagent for the formation of a carbon-carbon bond.

10. A process for the preparation of an alcohol compound, said process comprising the step of reacting (i) an aldehyde or a ketone with (ii) an organozinc composition of any of claims 1 to 4.

11. A process for the preparation of a β,γ-unsaturated ketone, said process comprising the step of reacting (i) an activated carboxylic acid with (ii) an organozinc composition of any of claims 1 to 4.

12. The process of claim 11, wherein the activated carboxylic acid is selected from a carboxylic acid chloride and a carboxylic acid anhydride.

13. A process for coupling two organic compounds via formation of a carbon-carbon bond, said process comprising the step of reacting
(i) an organic compound containing at least one non-aromatic double bond and a substituent X³ bound to a carbon atom in allyl-position to the non-aromatic carbon-carbon double bond, wherein X³ is an electrophilic leaving group, and
(ii) an organozinc composition of any of claims 1 to 4.

14. The process of any of claims 10 to 13, which is carried out in the absence of a catalyst.

15. A process for the preparation of a compound comprising an aryl or heteroaryl moiety, which aryl or heteroaryl moiety carries a substituent containing a non-aromatic carbon-carbon double bond, said process comprising the step of carrying out a cross-coupling reaction between
(i) a compound comprising an aryl or heteroaryl moiety, which aryl or heteroaryl moiety carries an electrophilic substituent, and
(ii) an organozinc composition of any of claims 1 to 4,
in the presence of an organometallic compound acting as a catalyst for the cross-coupling reaction.
